# EUROPÄISCHE PATENTSCHRIFT

(11) **EP 2 762 177 B1**
(45) Veröffentlichungstag und Bekanntmachung des Hinweises auf die Patenterteilung: **09.08.2017**
(21) Anmeldenummer: 14164190.2
(22) Anmeldetag: 24.06.2010
(51) Int. Cl.: A61M 1/00

(54) **Vorrichtung zur Unterdrucktherapie von Wunden**
Apparatus for low pressure treatment of wounds
Dispositif de traitement par dépressurisation de plaies

(30) Priorität: 06.07.2009 DE 102009031992
(43) Veröffentlichungstag der Anmeldung: 06.08.2014
(62) Teilanmeldung aus: 10734669.4
(73) Patentinhaber: Paul Hartmann AG, 89522 Heidenheim (DE)
(72) Erfinder: Eckstein, Axel, 89522 Heidenheim (DE); Fink, Ulrich, 79379 Müllheim (DE); Croizat, Pierre, 89542 Herbrechtingen (DE)
(74) Vertreter: Eibl, Christian

(56) Entgegenhaltungen:
- WO-A1-2005/123170
- WO-A1-2007/051599
- WO-A2-2007/030598
- WO-A2-2009/060097
- DE-U1-202004 017 052
- DE-U1-202004 018 245
- GB-A- 2 415 908

## Beschreibung

Die Erfindung betrifft eine neue Vorrichtung zur Unterdrucktherapie von Wunden umfassend ein luftundurchlässiges Abdeckmaterial zum luftdichten Verschließen der Wunde und der Wundumgebung, und ein Mittel zur funktionellen Verbindung des Wundraums mit einer außerhalb des Abdeckmaterials befindlichen Unterdruckquelle, so dass ein Unterdruck im Wundraum herstellbar ist und Flüssigkeiten aus dem Wundraum absaugbar sind, siehe z.B. WO2009/060097. Unter einer Wunde wird die Trennung des Zusammenhangs von Geweben der Körperhülle bei Menschen oder Tieren verstanden. Sie kann mit einem Verlust an Substanz verbunden sein.

Vorrichtungen zur Unterdrucktherapie von Wunden sind im Stand der Technik bekannt.

So beschreibt beispielsweise die WO1993/009727 eine Vorrichtung zur Förderung der Wundheilung durch die Applikation von Unterdruck auf dem die Wunde aufweisenden und die Wunde umgebenden Hautbereich. Die Vorrichtung gemäß WO1993/009727 umfasst eine Vakuumeinrichtung zur Erzeugung des Unterdrucks, eine als Dichtungseinrichtung bezeichnete luftdichte Abdeckung der Wunde, welche mit der Vakuumeinrichtung in einer funktionellen Verbindung steht, sowie eine als Schutzeinrichtung bezeichnete Wundauflage zur Positionierung an der Wunde innerhalb der Dichtungseinrichtung. Bei der Schutzeinrichtung handelt es sich um einen offenzelligen Polymer-Schaumstoff, beispielsweise um Polyester-Schaum. Ausweislich der Beschreibung von WO1993/009727 kann durch die Anwendung der Unterdrucktherapie die Wundheilung unterschiedlich Typen von Wunden wie beispielsweise Brandwunden, Druckwunden oder Platzwunden beschleunigt werden.

Während der Behandlung kann der Unterdruck bis zum Verbandwechsel kontinuierlich aufrechterhalten werden. Alternativ kann der Unterdruck während alternierender Zyklen appliziert werden, oder es können Zyklen unterschiedlich starken Unterdrucks zur Anwendung gebracht werden.

Geräte zur Unterdrucktherapie von Wunden sind kommerziell erhältlich, beispielsweise das V.A.C.^{®}-Gerät der Firma KCl. Bei kommerziell erhältlichen Geräten wird oftmals eine Wundauflage eingesetzt, welche einen offenzelligen Polymer-Schaumstoff, wie beispielsweise Polyvinylalkohol (PVA) oder Polyurethan (PU), enthält.

Schaumauflagen werden abhängig vom angelegten Unterdruck unterschiedlich stark komprimiert. Dadurch kann eine Verengung der für den Abtransport des Wundexsudats nötigen Durchgänge entstehen. Weiterhin kann bei längerer Anwendung von Polyvinylalkohohl oder Polyurethanschaum in der Unterdrucktherapie eine Verklebung des Schaums mit dem Wundgrund auftreten. Verklebter Schaum muss beim Wechseln des Verbandes aufwändig entfernt werden, beispielsweise durch Spülen mit Ringerlösung. In den Schaum eingewachsenes Gewebe kann beim Verbandwechsel zu einer Gewebetraumatisierung und damit zur Störung der Wundheilung führen.

Neben der Verwendung von offenzelligem Polymer-Schaumstoff wurden andere Materialien zur Herstellung von Wundauflagen für die Unterdrucktherapie von Wunden beschrieben. WO2001/89431 beschreibt eine Kollagen-Matrix als Wundauflage für die Unterdrucktherapie von Wunden.

GB2415908 beschreibt die Verwendung faserförmigen Materials, welches auch bioresorbierbar sein kann, in Wundauflagen für die Unterdrucktherapie von Wunden.

WO2006/52839 beschreibt die Verwendung trockenen faserförmigen Materials oder einer faserförmigen Mischung als Wundkontaktschicht für die Unterdrucktherapie von Wunden. Während der Unterdrucktherapie bildet das faserförmige Material durch Aufnahme von Wundexsudat ein Gel. Zwischen der aus dem Gel gebildeten Wundkontaktschicht und dem Abdeckmaterial kann sich eine Schaum-Schicht befinden. Die Schaum-Schicht kann das Abdeckmaterial ersetzen und die Funktion eines luftundurchlässigen Abdeckmaterials übernehmen.

In einer Ausführungsform der WO 2006/52839 kann die Wunde gespült werden. Dies wird durch einen zusätzlich in den Wundraum eingebrachten Schlauch erreicht, durch den eine Spülflüssigkeit in die Wunde gepumpt werden kann. Die Spülflüssigkeit wird aus dem Wundraum durch den Schlauch entfernt, welcher auch zur Erzeugung und Aufrechterhaltung des Unterdrucks dient. Der den Unterdruck erzeugenden Pumpe muss eine Flüssigkeitsfalle vorgeschaltet sein. Die Spülvorrichtung bedarf eines komplizierten Aufbaus, welcher sich nur in stationären Anlagen realisieren lässt.

WO2005/123170 offenbart Wundauflagen für die Unterdrucktherapie, mittels denen unerwünschte Substanzen inaktiviert oder aus dem Wundraum entfernt werden sollen und/oder erwünschte Substanzen, welche im Wundraum vorhanden sind konzentriert werden sollen. Als für den erwünschten Zweck geeignet Bestandteile von Wundauflagen schlägt die WO2005/123170 eine Vielzahl von zunächst trockenen Polymeren vor, die durch Wasseraufnahme aus dem Wundexsudat ein Gel bilden können. Die gelbildenden Polymere ermöglichen es auf die Verwendung einer Flüssigkeitsfalle zwischen Verband und Pumpe zu verzichten, da das Wundexsudat im Polymer zurückgehalten wird und nicht in den Absaugschlauch eintritt. Als geeignete Polymere werden beispielsweise quervernetzte Polyacrylat-Gele und superabsorbierende Gele vorgeschlagen.

WO2006/048246 beschreibt einen Mehrkomponentenverband zur Unterdruckbehandlung von Wunden, welcher ein superabsorbierendes Polymer aufweist. Das superabsorbierende Polymer kann von einer flüssigkeitsdurchlässigen Hülle eingefasst sein und einen trockenen Absorptionskörper bilden, der in den Wundraum eingelegt wird. Während der Unterdruckbehandlung vergrößert sich das Volumen des Absorptionskörpers durch die Aufnahme von Flüssigkeit aus dem Wundexsudat.

Auch WO2006/056294 und WO2006/048240 beschreiben Drainagevorrichtungen, welche ein superabsorbierendes Polymer aufweisen, zur Unterdruckbehandlung von Wunden. Zwischen Absorptionskörper und Wundoberfläche kann sich ein schleimhautfreundliches Schutzelement befinden. Während der Unterdruckbehandlung vergrößert sich die Querschnittsfläche des Absorptionskörpers sehr stark und nähert sich einer Kreisform an. Eine Vergrößerung des Anfangsvolumens des Absorptionskörpers ist ausweislich der Beschreibung der WO2006/056294 ausdrücklich erwünscht, um so die absorbierten Wundsekrete bis zur Entfernung des Absorptionskörpers aus dem Wundraum innerhalb des Absorptionskörpers zurückzuhalten. Die erfindungsgemäße starke Volumenzunahme wird durch die Applikation eines trockenen Absorptionskörpers in die Wunde ermöglicht.

Der vorliegenden Erfindung liegt die Aufgabe zugrunde die Unterdruckbehandlung von Wunden weiter zu verbessern und die Nachteile des Standes der Technik zu überwinden. Insbesondere liegt der vorliegenden Erfindung die Aufgabe zugrunde, Vorrichtungen und Verfahren zur Unterdruckbehandlung von Wunden zur Verfügung zu stellen, mit denen eine Behandlung möglichst wirksam und möglichst schonend durchgeführt werden kann.

Die Erfindung löst die Aufgabe mit einer Vorrichtung zur Unterdrucktherapie von Wunden mit den Merkmalen von Anspruch 1, sowie ein gebrauchsfertiges Set zur Unterdruck-Wundbehandlung gemäß Anspruch 13 Überraschend wurde nun gefunden, dass eine Vorrichtung zur Unterdrucktherapie von Wunden, umfassend ein luftundurchlässiges Abdeckmaterial zum luftdichten Verschließen der Wunde und der Wundumgebung, ein Mittel zur funktionellen Verbindung des Wundraums mit einer außerhalb des Abdeckmaterials befindlichen Unterdruckquelle, so dass ein Unterdruck im Wundraum herstellbar ist und Flüssigkeiten aus dem Wundraum absaugbar sind und mindestens einen aktivierten Saug-/Spülkörper, welcher mindestens ein Superabsorbierendes Polymer enthält, zum Einbringen in den Zwischenraum, welcher durch Wundoberfläche und Abdeckmaterial gebildet wird, zu einer sehr wirksamen und sehr schonenden Behandlung von Wunden besonders geeignet ist.

Die neue erfindungsgemäße Vorrichtung zeichnet sich insbesondere dadurch aus, dass sie mindestens einen aktivierten Saug-/Spülkörper, welcher mindestens ein Superabsorbierendes Polymer enthält, umfasst.

Unter "Superabsorbierendes Polymer" wird allgemein ein Wasser-unlösliches quellbares Polymer verstanden, welches ein Vielfaches seines Eigengewichtes an Flüssigkeit wie zum Beispiel Wasser, Salzlösungen oder Körperflüssigkeiten, aufnehmen kann. Die Flüssigkeitsaufnahme führt zur Ausbildung eines Hydrogels. Die Aufnahmefähigkeit für reines Wasser ist typischerweise höher als die Aufnahmefähigkeit für salzhaltige Flüssigkeit. Im Rahmen dieser Erfindung wird unter dem Ausdruck "Superabsorbierendes Polymer" insbesondere ein Polymer verstanden, welches gemäß der Standard-Testmethode WSP 240.2 (05) einen w-Wert ("free swell capacity") von mindestens 10 g/g, vorzugsweise mindestens 20 g/g aufweist. Die Testmethode WSP 240.2 (05) zur Bestimmung des w-Wertes ist in "Standard Test Methods for the Nonwovens and Related Industries", Ausgabe 2008 beschrieben (herausgegeben von "EDANA, International Association Serving the Nonwovens and Related Industries", Cary, NC, USA und "INDA, Association of the Nonwovens Fabrics Industry", Brüssel, Belgien).

WSP 240.2 (05) nach EDANA ist eine Standard-Testmethode zur Ermittlung des w-Wertes ("free swell capacity") von Superabsorbierendem Polyacrylat-Pulver. Nach WSP 240.2 (05) wird die freie Aufnahmekapazität für eine 0,9 Gewichtsprozent Kochsalzlösung bestimmt. Im Zusammenhang mit der vorliegenden Erfindung wird die Bestimmung des w-Wertes von Superabsorbierendem Material, bei dem es sich nicht um Polyacrylat-Pulver handelt, auf entsprechende Weise durchgeführt.

Das Superabsorbierende Polymer kann vorzugsweise in Form von Partikeln oder Fasern vorliegen.

Unter einem aktivierten Saug-/Spülkörper wird erfindungsgemäß ein Saug-/Spülkörper verstanden, welcher ein Superabsorbierendes Polymer enthält und welcher mittels einer wässrigen Aktivierungslösung aktiviert wurde. Unter "wässriger Aktivierungslösung" wird eine Flüssigkeit mit einem Wassergehalt von mindestens 50 Gewichtsprozent verstanden. Infolge der Aktivierung enthält der aktivierte Saug-/Spülkörper mindestens 500 Gewichtsprozent einer wässrigen Aktivierungslösung, bezogen auf das Gewicht des trockenen Saug-/Spülkörpers. Das in einem aktivierten Saug-/Spülkörper enthaltene mindestens eine Superabsorbierende Polymer liegt infolge der Aktivierung wenigstens teilweise in einem gequollenem Zustand vor.

In einer weiteren Ausführungsform enthält der aktivierte Saug-/Spülkörper mindestens 600 Gewichtsprozent, bevorzugt mindestens 800 Gewichtsprozent, besonders bevorzugt mindestens 1000 Gewichtsprozent einer wässrigen Aktivierungslösung bezogen auf das Gewicht des trockenen Saug-/Spülkörpers.

Weiterhin enthält der aktivierte Saug-/Spülkörper vorzugsweise weniger als 5000 Gewichtsprozent, besonders bevorzugt weniger als 3500 Gewichtsprozent, ganz besonders bevorzugt weniger als 2500 Gewichtsprozent einer wässrigen Aktivierungslösung.

In einer besonders bevorzugten Ausführungsform enthält der aktivierte Saug-/Spülkörper diejenige Menge einer wässrigen Aktivierungslösung, die seiner maximalen Aufnahmekapazität für Ringerlösung entspricht. Die maximale Aufnahmekapazität für Ringerlösung kann entsprechend der vorgenannten Testmethode WSP 240.2 (05) bestimmt werden, wobei jedoch
a) anstelle der in WSP 240.2 (05) verwendeten Kochsalzlösung Ringerlösung eingesetzt wird und
b) anstelle der in einer Hülle versiegelten Testsubstanz ("Bag" gemäß Absatz 6.1 von WSP 240.2 (05)) der erfindungsgemäße Saug-/Spülkörper eingesetzt wird.

Die maximale Aufnahmekapazität entspricht der nach diesem Verfahren gravimetrisch ermittelten Gewichtsdifferenz zwischen trockenem Saug-/Spülkörper und aktiviertem Saug-/Spülkörper, wobei eine Abweichung der Gewichtsdifferenz um 15 % nach oben oder unten umfasst ist.

Der aktivierte Saug-/Spülkörper enthält mindestens ein Superabsorbierendes Polymer und gegebenenfalls einen oder mehrere Hilfs- oder Trägerstoffe. Er kann von einer Hülle umgeben sein, welche zumindest in bestimmten Bereichen der Hülle den Durchtritt von Flüssigkeiten erlaubt.

Vom den aus dem Stand der Technik bekannten trockenen Absorptionskörpern, welche bisher für die Unterdrucktherapie eingesetzt wurden, unterscheidet sich der erfindungsgemäße Saug-/Spülkörper darin, dass es sich um einen aktivierten Saug-/Spülkörper handelt. Überraschenderweise können durch die Aktivierung des Saug-/Spülkörpers vorteilhafte Eigenschaften bei der Unterdruckbehandlung von Wunden erreicht werden.

Ein Vorteil des aktivierten Saug-/Spülkörper als Bestandteil der erfindungsgemäßen Vorrichtung besteht darin, dass er eine weiche Wundauflage für die Unterdrucktherapie von Wunden bereitstellt.

Ein weiterer Vorteil des aktivierten Saug-/Spülkörper als Bestandteil der erfindungsgemäßen Vorrichtung besteht darin, dass eine gleichmäßige Druckverteilung auf den Wundgrund gewährleistet werden kann.

Durch die Bereitstellung einer weichen Wundauflage und durch die gleichmäßige Druckverteilung kann die Unterdruckbehandlung schonend und besonders wirksam durchgeführt werden.

Ein weiterer Vorteil des aktivierten Saug-/Spülkörper als Bestandteil der erfindungsgemäßen Vorrichtung besteht darin, dass ein Verkleben und/oder Verwachsen des Wundgrundes mit der Wundauflage weitgehend vermieden werden kann. Dies ist vorteilhaft, da keine zusätzliche Reinigung der Wunde nach dem Abnehmen des Unterdruckverbandes erfolgen muss. Der Verbandswechsel kann schonender und schneller durchgeführt werden. Eine Traumatisierung der Wunde beim Verbandswechsel kann vermieden werden. Dies erhöht die Wirksamkeit der Wundbehandlung.

Ein weiterer Vorteil des aktivierten Saug-/Spülkörper als Bestandteil der erfindungsgemäßen Vorrichtung besteht darin, dass unmittelbar nach Beginn der Unterdruckbehandlung ein die Heilung unterstützender hydroaktiver Effekt vorliegt. Unter einem hydroaktiven Effekt wird hier verstanden, dass die Wunde in einem feuchten Milieu gehalten wird und nicht austrocknet. Wie schon lange bekannt ist (siehe beispielsweise GD Winter (1962) in Nature 193, S. 293-294) führt eine Austrocknung der Wunde zu Schorfbildung, die sehr rasch, beispielsweise innerhalb von 24 Stunden, eintreten kann. Als Folge der Schorfbildung wird die Wundheilung verlangsamt. Der hydroaktive Effekt dagegen unterdrückt die Schorfbildung und fördert das Wachstum des Granulationsgewebes. Ein früh einsetzender hydroaktiver Effekt kann sich deshalb sehr günstig auf den Verlauf der Wundheilung auswirken.

Der hydroaktive Effekt bei Verwendung des erfindungsgemäßen aktivierten Saug-/Spülkörpers lässt sich durch die Beobachtung erklären, dass der aktivierte Saug-/Spülkörper während einer Unterdruckbehandlung Aktivierungslösung abgeben kann. Infolge der Abgabe von Aktivierungslösung ist der aktivierte Saug-/Spülkörper zudem in der Lage gegebenenfalls im Austausch Wundexkret zu absorbieren. Insbesondere kann der aktivierte Saug-/Spülkörper Bakterien und schädliche Bestandteile des Wundexsudates aufnehmen. Die Absorption von Bakterien und schädlicher Bestandteile des Wundexsudates begünstigt die Wundheilung zusätzlich und wirkt Wundinfektionen entgegen.

Insbesondere kann durch den aktivierten Saug-/Spülkörper bei der Unterdruckbehandlung eine vorteilhafte Spülwirkung erzielt werden. Dies ermöglicht die Reinigung der Wunde während der Behandlung und ist vorteilhaft für die Wundheilung. Die Spülwirkung ist während der so genannten Reinigungsphase zu Beginn des Wundheilungsprozesses besonders vorteilhaft. Eine ausgeprägte Spülwirkung kann besonders dann erreicht werden, wenn Unterdruck wechselnder Stärke appliziert wird.

In einer bevorzugten Ausführungsform stellt die Erfindung eine Vorrichtung zur Unterdrucktherapie von Wunden zur Verfügung, wobei es sich bei dem Superabsorbierenden Polymer um einen Partikel oder um eine Faser handelt.

In einer weiteren bevorzugten Ausführungsform stellt die Erfindung eine Vorrichtung zur Unterdrucktherapie von Wunden zur Verfügung, wobei es sich bei dem Superabsorbierenden Polymer um ein Superabsorbierendes Polyacrylat handelt.

Hierbei wird im Rahmen der vorliegenden Erfindung unter Polyacrylat ein synthetisches Polymer verstanden, umfassend als Monomer (M1) Acrylsäure (2-Propensäure, CH₂=CH-CO₂H) und/ oder ein Salz hiervon. Der Monomeranteil beträgt insbesondere mehr als 70 Gewichtsprozent Acrylsäure und/ oder eines Salzes hiervon (bezogen auf das Gesamtgewicht des Polyacrylats). Vorzugsweise weisen erfindungsgemäße Polyacrylate einen Monomeranteil von mehr als 80 Gewichtsprozent Acrylsäure und/oder eines Salzes hiervon und ganz besonders bevorzugt mehr als 95 Gewichtsprozent Acrylsäure und/oder eines Salzes hiervon bezogen auf das Gesamtgewicht des Polyacrylats auf. Dabei kann das Polyacrylat als Homopolymer, Copolymer oder Blockpolymer vorliegen. Falls das Polyacrylat als Copolymer oder Blockpolymer vorliegt, so liegt in jedem Fall der Monomeranteil des Monomers M1 in dem Polymer bei mehr als 70 Gewichtsprozent, insbesondere bei mehr als 80 Gewichtsprozent und ganz besonders bevorzugt bei mehr als 95 Gewichtsprozent bezogen auf das Gesamtgewicht des Polyacrylats. In diesen copolymeren Polyacrylaten oder blockpolymeren Polyacrylaten können neben dem Monomer M1 als Comonomere M2 insbesondere α,β-ungesättigte Ether (Vinylether), α,β-ungesättigte Carbonsäuren oder α,β-ungesättigte Carbonsäureester (Vinylester) enthalten sein. Von den Comonomeren □M2 der α,β-ungesättigten Carbonsäuren werden besonders Methacrylsäure (2-Methylpropensäure), Ethacrylsäure (2-Ethylpropensäure), Crotonsäure (2-Butensäure), Sorbinsäure (*trans-trans-*2,4-Hexadiensäure), Maleinsäure (*cis*-2-Butendisäure) oder Fumarsäure (*trans*-2-Butendisäure) bevorzugt. Es kann jedoch auch vorgesehen sein, dass das Polyacrylat aus a) einem Homopolymer aus Acrylsäure und/oder b) einem Copolymer aus i) Acrylsäure und einem Salz der Acrylsäure, ii) aus Methacrylsäure und einem Salz der Methacrylsäure oder iii) aus Acrylsäure und Methacrylsäure und deren Salze besteht. Weiterhin kann jedoch auch vorgesehen sein, dass es sich bei dem Polyacrylat um eine Mischung von verschiedenen Polyacrylaten handelt.

Hierbei können insbesondere die α,β-ungesättigten Carbonsäuren als auch die Acrylsäure in neutralisierter Form als Salz, in nicht-neutralisierter Form als freie Säure oder in Mischungen hiervon vorliegen. Insbesondere haben sich Polyacrylate, die aus Acrylsäure und Salzen der Acrylsäure aufgebaut sind als besonders geeignet gezeigt. Dabei sind insbesondere Alkalimetall- oder Erdalkalimetallsalze hervorzuheben. Insbesondere zeigten sich Polyacrylate, die aus Homopolymeren und/oder aus Copolymeren bestehen, die als Monomere Acrylsäure und/ oder Natrium- oder Kaliumacrylat umfassen, als besonders für den erfindungsgemäßen Saug-/Spülkörper geeignet.

Weiterhin hat sich gezeigt, dass sich Polyacrylate aus der Gruppe der vernetzten und/ oder quervernetzten und/ oder oberflächenvernetzten Polyacrylate besonders geeignet sind. Diese Polyacrylate umfassen vorzugsweise a) ein Homopolymer, das aus den Monomeren M1 besteht und mittels eines Vernetzers vernetzt und/ oder quervernetzt ist, und/ oder b) ein Copolymer, das aus den Monomeren M1 und M3 besteht, wobei das Monomer M1 Acrylsäure und/ oder ein Salz hiervon ist und das Monomer M3 aus der Gruppe der Vernetzer gewählt wird. Das heißt, dass diese Polyacrylate ein mittels eines Vernetzers nachträglich vernetztes Polyacrylat und/ oder ein Polyacrylat umfassen, das aus Acrylsäure und/ oder einem Salz hiervon und einem Vernetzer copolymerisiert ist. Insbesondere hat sich gezeigt, dass vernetze und/ oder quervernetzte Polyacrylate, die als Vernetzer Verbindungen V1 enthalten, die mindestens zwei ethylenisch ungesättigte Gruppen innerhalb eines Moleküls aufweisen, oder Verbindungen V2, die mindestens zwei funktionelle Gruppen aufweisen, die mit funktionellen Gruppen der Acrylsäure oder/ und eines Salzes hiervon in einer Kondensationsreaktion, in einer Additionsreaktion oder in einer Ringöffnungsreaktion reagieren können, oder Verbindungen V3, die mindestens eine ethylenisch ungesättigte Gruppe und mindestens eine funktionelle Gruppe, die mit funktionellen Gruppen der Acrylsäure und/ oder eines seiner Salze und/ oder den α,β-ungesättigten Comonomere in einer Kondensationsreaktion, in einer Additionsreaktion oder in einer Ringöffnungsreaktion reagieren kann, besonders für die erfindungsgemäßen Saug-/Spülkörper geeignet sind. Dabei wird durch die Verbindungen V1 eine Vernetzung der Polymere durch die radikalische Polymerisation der ethylenisch ungesättigten Gruppen des Vernetzermoleküls mit den monoethylenisch ungesättigten Monomeren Acrylsäure und/ oder eines seiner Salze und/ oder einer der α,β-ungesättigten Comonomere erreicht, während bei den Verbindungen V2 eine Vernetzung der Polymere durch Kondensationsreaktion der funktionellen Gruppen mit den funktionellen Gruppen der Acrylsäure und/ oder eines seiner Salze oder einer der α,β-ungesättigten Comonomere erreicht wird. Bei den Verbindungen V3 erfolgt dementsprechend eine Vernetzung des Polymers sowohl durch radikalische Polymerisation der ethylenisch ungesättigten Gruppe als auch durch Kondensationsreaktion zwischen der funktionellen Gruppe des Vernetzers und den funktionellen Gruppen der Monomeren. Bevorzugte Verbindungen V1 sind Polyacrylsäureester oder Polymethacrylsäureester, die beispielsweise durch die Umsetzung eines Polyols, wie beispielsweise Ethylenglykol (1,2-Ethandiol), Propylenglykol (1,2-Propandiol), Trimethylolpropan (2-Ethyl-2-hydroxymethyl-1,3-propandiol), 1,6-Hexandiol, Glycerin (1,2,3-Propantriol), Pentaerythrit (2,2-Bis(hydroxymethyl)propan-1,3-diol), Polyethylenglykol (HO-(CH₂ - CH₂- O)ₙ-H mit n = 2 bis 20) mit n = 1 bis 20), Polypropylenglykol (HO-(CH(CH₃) - CH₂- O)ₙ-H mit n = 2 bis 20), eines Aminoalkohols, eines Polyalkylenpolyaminens, wie beispielsweise Diethylentriamin oder Triethylentetraamin, oder eines alkoxylierten Polyols mit Acrylsäure oder Methacrylsäure gewonnen werden. Bevorzugt kann es sich bei dem vernetzten Polyacrylaten auch um ein Polyacrylat handeln, das mittels einer Verbindung V1 vernetzt ist, die ein Di-, Tri- oder Tetraester der Polyacrylsäure oder Polymethacrylsäure ist, der durch die Umsetzung eines alkoxylierten Polyols, insbesondere eines ethoxylierten Polyols, insbesondere ethoxyliertes Ethylenglycol, ethoxyliertes Propylenglycol, ethoxyliertes Trimethylolpropan, ethoxyliertes 1,6-Hexandiol oder ethoxyliertes Glycerin, mit einer durchschnittlichen Anzahl an Ethylenoxideinheiten n pro Hydroxygruppe von n = 1 bis 10 mit Acrylsäure oder Methacrylsäure gewonnen werden. Als Verbindungen V1 sind des Weiteren Polyvinylverbindungen, Polyallylverbindungen, Polymethylallylverbindungen, Acrylsäureester oder Methacrylsäureester einer Monovinylverbindung, Acrylsäureester oder Methacrylsäureester einer Monoallylverbindung oder Monomethylallylverbindung, vorzugsweise der Monoallylverbindungen oder Monomethylallylverbindungen eines Polyols oder eines Aminoalkohols, bevorzugt. In diesem Zusammenhang wird auf die DE 195 43 366 und die DE 195 43 368 verwiesen.

Die in der Erfindung genannten Superabsorbierenden Polymere aus Polyacrylat zeichnen sich durch eine ausgezeichnete Absorptionsfähigkeit, sowie Fähigkeit zum Austausch von Flüssigkeiten aus und sind deshalb für die erfindungsgemäße Vorrichtung besonders geeignet. Die Polymere ermöglichen weiterhin einen Spüleffekt, da die Affinität des Polyacrylats zum proteinhaltigen Wundexsudat höher ist als die Affinität zur salzhaltigen Aktivierungslösung (beispielsweise Ringerlösung). Damit kann erreicht werden, dass Wundexsudat die Aktivierungslösung aus dem Wundkissen verdrängt.

Weitere Vorteile der vorgenannten Superabsorber sind eine weiche Materialstruktur, gute physiologische Verträglichkeit, geringe Toxizität und hohe Sicherheit.

In einer bevorzugten Ausführungsform handelt es sich bei dem Superabsorbierendem Polymer um ein Gemisch von Polyacrylatpartikeln, wobei die Polyacrylatpartikel die im Zusammenhang mit der Erfindung aufgeführte chemische Zusammensetzung aufweisen. Dabei kann auch vorgesehen sein, dass das Partikelgemisch Polyacrylatpartikel enthält, die sich hinsichtlich der Polyacrylate voneinander unterscheiden, d.h., dass das Partikelgemisch mindestens zwei voneinander verschiedene Polyacrylatpartikelsorten umfasst.

Die Polyacrylate können sich beispielsweise hinsichtlich des Neutralisationsgrades, des Vernetzungsgrades, des Vernetzungsmittels und/ oder der Copolymere unterscheiden.

Im einfachsten Fall können jedoch auch Polyacrylatpartikel verwendet werden, die hinsichtlich ihres strukturellen Aufbaus gleich sind und lediglich in den angegebenen Partikelgrößen verschieden sind. Insbesondere kann auch vorgesehen sein, dass sich die Polyacrylatpartikel eines ersten Größenbereichs a) von den Poyacrylatpartikeln eines zweiten Größenbereichs b) unterscheiden.

Es kann jedoch auch vorgesehen sein, dass die Partikel des ersten oder des zweiten Partikelgrößenbereichs aus verschiedenen Polyacrylatpartikeln besteht, d.h., dass beispielsweise die Partikel des ersten Größenbereichs erste Polyacrylatpartikel und zweite, von den ersten verschiedene Polyacrylatpartikel enthält, wobei beide Sorten Polyacrylatpartikel umfassen, die innerhalb des jeweiligen Größenbereichs liegen.

Dabei soll im Zusammenhang mit der vorliegenden Erfindung unter einem Partikelgemisch ein Gemenge verstanden sein, deren einzelne Bestandteile (Partikel) räumlich neben einander, teilweise durchmischt, vollständig durchmischt oder räumlich getrennt voneinander vorliegen können, wobei das Gemisch in jedem Fall als Bestandteil eines Saug-/Spülkörpers anzusehen ist. Insbesondere können dabei die Partikel der einzelnen Partikelgrößenbereiche auch räumlich nebeneinander, teilweise durchmischt, vollständig durchmischt oder räumlich getrennt voneinander vorliegen.

Damit umfasst der aktivierte Saug-/Spülkörper ein Partikelgemisch mit einer definierten Menge einer ersten Partikelfraktion mit einer definierten Partikelgröße und einer zweiten Menge einer zweiten Partikelfraktion mit einer von der ersten Partikelgröße verschiedenen zweiten Partikelgröße, wobei jede Partikelfraktion Polyacrylatpartikel enthält. Hierbei können sich die Polyacrylatpartikel eines Größenbereichs aus Partikeln gleicher oder unterschiedlicher Größe zusammensetzen, wobei die Partikelgröße(n) innerhalb des Bereichs liegt/ liegen. Im Zusammenhang mit der vorliegenden Erfindung wird dabei die Angabe einer Menge Partikel insbesondere eines einzelnen Größenbereichs - falls nicht anders angegeben - immer in Gewichtsprozent bezogen auf die Gesamtmenge an Partikeln angegeben.

Die Partikelgröße der trockenen Partikel wird im Zusammenhang mit der vorliegenden Erfindung analog zum Standardtest WSP 220-2-(05) bestimmt, wobei die Siebe (Durchmesser 200 mm) den Angaben entsprechende Lochgrößen aufweisen. Der Standardtest WSP 220-2-(05) ist in "Standard Test Methods for the Nonwovens and Related Industries", Ausgabe 2008 beschrieben. Darüber hinaus können auch Siebe mit anderen Lochgrößen wie z.B. 125 µm, 160 µm, 630 µm, 900 µm, und 1500 µm verwendet werden. Hierbei werden trockene Polyacrylatpartikel mit einem Feuchtigkeitsgehalt von weniger als 5 Gewichtsprozent Wasser bezogen auf das Gesamtgewicht der Partikel zugrunde gelegt, wobei der Feuchtigkeitsgehalt gemäß WSP 230.02 (05) bestimmt wird. Der Standardtest WSP 230-2-(05) ist in "Standard Test Methods for the Nonwovens and Related Industries", Ausgabe 2008 beschrieben.

Die Bestimmung der Partikelgröße gemäß der vorstehend bezeichneten Methode sollte vor der Herstellung des die Partikel enthaltenden Saug-/Spülkörpers durchgeführt werden. Die Bestimmung der Partikelgröße kann nicht an einem bereits aktivierten Saug-/Spülkörper erfolgen, da sich die Testmethode WSP 230-2-(05) auf Partikel mit einem Feuchtigkeitsgehalt von weniger als 5 Gewichtsprozent Wasser bezieht.

Aus der WO2009/68249 ist bekannt, dass Polyacrylatpartikel Proteasen sowohl inhibieren als auch durch direkte Bindung kompartmentieren und damit dem Wundexsudat bzw. der Wunde entziehen können. Ein Überschuss an insbesondere Metallo-Proteasen ist insbesondere in der Granulationsphase der Wundheilung störend. Bei einer Ausführungsform der erfindungsgemäßen Vorrichtung, welche einen aktivierten Saug-/Spülkörper mit Superabsorbierendem Polyacrylat enthält, können die Polyacrylatpartikel ein Überschuss an Metallo-Proteasen in chronischen Wunden derart abfangen und/oder inhibieren, dass ein natürlicher Heilungsverlauf stattfinden kann. Hierfür sind ausweislich der WO2009/68249 Polyacrylatpartikel mit einer Partikelgröße x mit x ≤ 300 µm besonders gut geeignet. Partikel dieser Größe hemmen die Aktivität von wundheilungsschädlichen Proteasen, insbesondere Metalloproteasen in einer Wunde besonders wirkungsvoll. Polyacrylatpartikel mit einer Partikelgröße x mit x ≤ 300 µm sind jedoch zur Aufnahme von Wundexsudat weniger gut oder gar nicht geeignet, da diese Partikel im Vergleich zu Partikeln mit einer Partikelgröße x mit x > 300 µm eine sehr viel geringere Menge an wässrigen Flüssigkeiten absorbieren oder halten können. Es ist deshalb vorteilhaft, wenn der aktivierte Saug-/Spülkörper ein Gemisch von Polyacrylatpartikeln enthält, bei dem sowohl Partikel enthalten sind, welche aufgrund Ihrer Größe Proteasen inhibieren und kompartimentieren können, als auch Partikel welche aufgrund Ihrer Größe ein hohes Absorptionsvermögen aufweisen.

Bei einer besonders bevorzugten Ausführungsform der erfindungsgemäßen Vorrichtung zur Unterdrucktherapie von Wunden handelt es sich deshalb bei dem Superabsorbierenden Polymer um ein Partikelgemisch von Polyacrylatpartikeln unterschiedlicher Größe, dadurch gekennzeichnet, dass das Partikelgemisch
a) 5 bis 100 Gewichtsprozent Partikel mit einer Partikelgröße x mit x ≤ 300 µm und
b) 0 bis 95 Gewichtsprozent Partikel mit einer Partikelgröße x mit x > 300 µm enthält.

Insbesondere handelt es sich deshalb bei dem Superabsorbierenden Polymer um ein Partikelgemisch von Polyacrylatpartikeln unterschiedlicher Größe, dadurch gekennzeichnet, dass das Partikelgemisch
a) 5 bis 98 Gewichtsprozent Partikel mit einer Partikelgröße x mit x ≤ 300 µm als Mittel zur Hemmung von Proteasen in der Wunde und
b) 2 bis 95 Gewichtsprozent Partikel mit einer Partikelgröße x mit x > 300 µm als Mittel zur Auf- und/ oder Abgabe von wässrigen Lösungen enthält.

Ganz besonders bevorzugt handelt es sich deshalb bei dem Superabsorbierenden Polymer um ein Partikelgemisch von Polyacrylatpartikeln unterschiedlicher Größe, dadurch gekennzeichnet, dass das Partikelgemisch
a) 20 bis 80 Gewichtsprozent Partikel mit einer Partikelgröße x mit x ≤ 300 µm als Mittel zur Hemmung von Proteasen in der Wunde und
b) 20 bis 80 Gewichtsprozent Partikel mit einer Partikelgröße x mit x > 300 µm als Mittel zur Auf- und/ oder Abgabe von wässrigen Lösungen enthält.

Der aktivierte Saug-/Spülkörper kann neben dem Superabsorbierendem Polymer, bei dem es sich vorzugsweise um ein Superabsorbierendes Polyacrylat handelt, ein Trägermaterial umfassen, wobei das Trägermaterial ein hydrophiles Fasermaterial umfasst.

Hierbei können als hydrophiles Fasermaterial insbesondere wasserunlösliche Fasern aus Cellulose, insbesondere weitgehend delignifizierte technische Zellstofffasern, insbesondere Holzzellstofffasern, insbesondere einer Faserlänge von < 5 mm Verwendung finden. Das Fasermaterial kann auch hydrophiles Fasermaterial aus regenerierter Cellulose, Carboxymethylcellulose, Carboxyethylcellulose, Hydroxymethylcellulose oder Hydroxyethylcellulose enthalten. Es kann auch vorgesehen sein eine Fasermischung aus Cellulose-, regenerierter Cellulose, Carboxymethylcellulose-, Carboxyethylcellulose-, Hydroxymethylcellulose- oder Hydroxyethylcellulose-Fasern und Fasern aus Polyethylen, Polypropylen oder Polyester vorzusehen.

In einer besonders bevorzugten Ausführungsform umfasst der aktivierte Saug-/Spülkörper mindestens ein Superabsorbierendes Polyacrylat der vorstehend angegebenen Zusammensetzung, sowie als Trägermaterial eine Mischung aus Cellulosefasern und Polypropylenfasern.

Diese Fasern können in einem so genannten Air-laid-Verfahren zusammen mit dem Partikelgemisch zu einer Schicht verarbeitet sein.

In einer besonders bevorzugten Ausführungsform handelt es sich bei dem aktivierten Saug-/Spülkörper um ein umhülltes Airlaid, welches ein Superabsorbierendes Polyacrylat, Cellulose- und Polypropylenfasern umfasst.

Das Airlaid kann in dieser besonders bevorzugten Ausführungsform von einem textilen Material umhüllt sein, insbesondere kann die Hülle aus einem Interlock-Gestrick aus Polypropylenfasern bestehen. Ein mit Polypropylen umhülltes Airlaid, welches Superabsorbierenden Polyacrylat, Cellulose- und Polypropylenfasern enthält ist unter der Bezeichnung TenderWet^{®} kommerziell erhältlich (Paul Hartmann AG, Deutschland).

Einen für die erfindungsgemäße Vorrichtung besonders geeigneten weichen und plastisch verformbaren Saug-/Spülkörper erhält man insbesondere dann, wenn seine Hülle mindestens teilweise aus einer textilen Fläche besteht, welche in Längs-, Quer-, und Diagonalrichtung nicht-elastisch dehnbar ist. In diesem Zusammenhang sei auf die EP0594034B1 verwiesen.

Eine von mehreren geeigneten Methoden zur Verschweißung von Hüllen aus thermoplastischen Stoffen ist die Ultraschallverschweißung. Der aktivierte Saug-/Spülkörper kann beispielsweise eine runde, rechteckige oder ovale Form aufweisen. Es sind jedoch auch andere Formen vorstellbar.

Der aktivierte Saug-/Spülkörper kann unterschiedliche Mengen an Superabsorbierendem Polymer und Trägermaterial aufweisen. Vorzugsweise enthält der aktivierte Saug-/Spülkörper mindestens 10 Gewichtsprozent Polyacrylat (bezogen auf das Trägermaterial), wobei das Polyacrylat die im Zusammenhang mit der Erfindung aufgeführte Zusammensetzung aufweist. Besonders bevorzugt sind jedoch aktivierte Saug-/Spülkörper, die mindestens 20 Gewichtsprozent, insbesondere mindestens 25 Gewichtsprozent und ganz besonders bevorzugt mindestens 30 Gewichtsprozent Polyacrylat (bezogen das Trägermaterial) umfassen. Um den aktivierten Saug-/Spülkörper hinsichtlich der Auf- und Abgabe von wässrigen Flüssigkeiten nicht einzuschränken, sollte jedoch gewährleistet sein, dass der Gehalt an Polyacrylat in Bezug auf das Trägermaterial vorzugsweise nicht mehr als 90 Gewichtsprozent und insbesondere nicht mehr als 75 Gewichtsprozent beträgt.

Die vorliegende Erfindung stellt eine sehr wirkungsvolle und schonende Vorrichtung zur Unterdrucktherapie von Wunden zur Verfügung. Um diese Vorteile erzielen zu können ist es wesentlich, dass die Vorrichtung einen aktivierten Saug-/Spülkörper umfasst. Es ist deshalb unerlässlich, dass der aktivierte Saug-/Spülkörper mindestens 500 Gewichtsprozent, bezogen auf das Gewicht des trockenen Saug-/Spülkörpers, einer wässrigen Aktivierungslösung enthält.

Es ist jedoch besonders vorteilhaft, wenn der aktivierte Saug-/Spülkörper mindestens 500 Gewichtsprozent, bezogen auf das Gewicht des trockenen Saug-/Spülkörpers, einer wässrigen Aktivierungslösung, umfassend
mehr als 50 Volumenprozent Wasser
mindestens 5 mmol/l Natrium Ionen
enthält, mit der Maßgabe, dass es sich bei der Aktivierungslösung um eine synthetische Aktivierungslösung handelt.

In weiteren bevorzugten Ausführungsformen enthält der aktivierte Saug-/Spülkörper mindestens 600 Gewichtsprozent, bevorzugt mindestens 800 Gewichtsprozent, besonders bevorzugt mindestens 1000 Gewichtsprozent bezogen auf das Gewicht des trockenen Saug-/Spülkörpers, der vorgenannten synthetischen Aktivierungslösung.

Vorzugsweise enthält der aktivierte Saug-/Spülkörper weniger als 5000 Gewichtsprozent, besonders bevorzugt weniger als 3500 Gewichtsprozent, ganz besonders bevorzugt weniger als 2500 Gewichtsprozent der synthetischen Aktivierungslösung.

Synthetisch bedeutet im Zusammenhang mit der Erfindung, dass die Aktivierungslösung auf eine technische Weise hergestellt ist. Lösungen, die direkt und ohne weitere Bearbeitung aus einem lebenden System, wie einem Menschen oder einem Tier stammen, sind deshalb von der Aktivierungslösung ausgenommen. Insbesondere besteht die Aktivierungslösung nicht aus einer Lösung, welche direkt aus einer Wunde abgesondert wird, d.h. es handelt sich bei der Aktivierungslösung nicht um menschliches oder tierisches Wundexsudat.

Die Aktivierungslösung kann Substanzen enthalten, die biologischen Ursprungs sind, unter der Voraussetzung, dass diese Substanzen der synthetischen Aktivierungslösung bei der Herstellung zugesetzt werden. Mit Substanzen biologischen Ursprungs sind solche Substanzen gemeint, die menschlichen, tierischen, pflanzlichen, oder mikrobiellen Ursprungs sind und/oder in solchen Organismen (oder Teilen daraus) hergestellt wurden.

Ein mit der synthetische Aktivierungslösung aktivierter Saug-/Spülkörper weist eine weiche Struktur auf und gewährleistet dadurch eine gleichmäßige Druckverteilung. Die synthetische Aktivierungslösung ermöglicht den hydroaktiven Effekt und wirkt sich positiv auf das chemische Milieu in der Wunde aus.

Weiterhin wurde gefunden, dass durch die Verwendung eines Saug-/Spülkörper, welcher mindestens 500 Gewichtsprozent bezogen auf das Gewicht des trockenen Saug-/Spülkörpers, der vorgenannten synthetischen Aktivierungslösung enthält, ein Verkleben und /oder Verwachsen der Wundauflage mit dem Wundgrund reduziert werden kann.

In einer bevorzugten Ausführungsform enthält der aktivierte Saug-/Spülkörper mindestens 500 Gewichtsprozent, bezogen auf das Gewicht des trockenen Saug-/Spülkörpers, einer synthetischen Aktivierungslösung, umfassend mehr als 50 Volumenprozent Wasser, mindestens 5 mmol/l Natrium Ionen, mindestens 0,1 mmol/l Kalium Ionen, mindestens 0,1 mmol/l Calcium Ionen und mindestens 5 mmol/l Chlorid Ionen. Optional enthält die synthetische Aktivierungslösung weitere anorganische Kationen und/oder Anionen, gegebenenfalls organische Anionen, gegebenenfalls antimikrobielle Substanzen, sowie gegebenenfalls Zusätze bioorganischer Verbindungen. Der pH-Wert beträgt vorzugsweise 4 bis 9. Die Viskosität bei 20°C beträgt vorzugsweise zwischen 0,8 mPa s und 150 mPa s. Die Viskosität der Aktivierungslösung wurde mit einem Brookfield Viskosimeter bestimmt (Einheit: 1 Pa·s= 1 Ns/m²). Die in dieser bevorzugten Ausführungsform verwendete synthetische Aktivierungslösung ist hinsichtlich chemischer Zusammensetzung, pH-Wert und Viskosität besonders zur Aktivierung des Saug-/Spülkörper geeignet. Bei Verwendung eines mit der vorgenannten synthetischen Aktivierungslösung aktivierten Saug-/Spülkörper kann eine besonders effektive Unterdrucktherapie von Wunden durchgeführt werden. Dies beruht auf einer synergistischen Wechselwirkung zwischen der speziell ausgewählten synthetischen Aktivierungslösung und dem Superabsorbierenden Polymer.

In einer ganz besonders vorteilhaften und deshalb besonders bevorzugten Ausführungsform handelt es sich bei der synthetischen Aktivierungslösung um Ringerlösung.

Die bereits genannten Vorteile aktivierter Saug-/Spülkörper in der Unterdruckbehandlung treten bei einem Saug-/Spülkörper, welcher als synthetische Aktivierungslösung eine Ringerlösung enthält, noch stärker hervor. Unter einer Ringerlösung wird eine annähernd zum Blut iso-osmotische synthetische Lösung verstanden, welche Natriumchlorid, Kaliumchlorid und Calciumchlorid umfasst. Vorzugsweise enthält die Ringerlösung 147 mmol/l Natrium Ionen, 4,0 mmol/l Kalium Ionen, 3,0 mmol/l Calcium Ionen und 157 mmol/l Chlorid Ionen, wobei eine Abweichung der jeweiligen Ionenkonzentration vom angebenden Wert um 5 % nach oben oder unten möglich ist. Ringerlösung lässt sich beispielsweise herstellen, indem 8,6 g Natriumchlorid, 0,30 g Kaliumchlorid und 0,33 g Calciumchlorid in einem Liter destillierten Wasser aufgelöst werden.

Die vorteilhafte Wirkung der Ringerlösung bei der Unterdruckbehandlung von Wunden ist in der so genannten Reinigungsphase der Wundheilung besonders ausgeprägt.

Vorzugsweise wird der mit der synthetischen Aktivierungslösung aktivierte Saug-/Spülkörper abgepackt zur Verfügung gestellt, so dass dieser vor dem Anlegen des Verbandes nur noch entnommen zu werden braucht.

Besonders bevorzugt ist es, einen mit Ringerlösung aktivierten Saug-/Spülkörper abgepackt zur Verfügung zu stellen, so dass dieser vor dem Anlegen des Verbandes nur noch entnommen zu werden braucht. Weiterhin ist es besonders bevorzugt, den mit der synthetischen Aktivierungslösung aktivierten Saug-/Spülkörper in steriler Form abgepackt zu Verfügung zu stellen.

Erfindungsgemäß wird eine Vorrichtung zur Unterdrucktherapie von Wunden zur Verfügung gestellt, umfassend einen aktivierten Saug-/Spülkörper, wobei es sich bei dem Saug-/Spülkörper um einen volumenstabilen Saug-/Spülkörper handelt. Der volumenstabile Saug-/Spülkörper weist während der Unterdruckbehandlung keine Volumenzunahme auf. Stattdessen weist der volumenstabile Saug-/Spülkörper während der Unterdruckbehandlung eine Abnahme des Volumens auf. Die Abnahme des Volumens des Saug-/Spülkörper geht mit der Abnahme des Gewichtes des Saug-/Spülkörpers einher.

Die Volumenstabilität wird erhalten, indem der Saug-/Spülkörper mit einer wässrigen Aktivierungslösung, bevorzugt mit einer synthetischen Aktivierungslösung, insbesondere mit einer synthetischen Aktivierungslösung wie zuvor beschrieben, besonders bevorzugt mit Ringerlösung, aktiviert wird.

Dabei ist es vorteilhaft, dass der volumenstabile Saug-/Spülkörper mindestens 500 Gewichtsprozent, vorzugsweise 600 Gewichtsprozent, besonders bevorzugt 800 Gewichtsprozent der Aktivierungslösung, enthält. In einer weiteren vorteilhaften Ausführungsform enthält der volumenstabile Saug-/Spülkörper mindestens 1000 Gewichtsprozent der Aktivierungslösung. Vorzugsweise enthält der volumenstabile Saug-/Spülkörper weniger als 5000 Gewichtsprozent, besonders bevorzugt weniger als 3500 Gewichtsprozent, ganz besonders bevorzugt weniger als 2500 Gewichtsprozent der Aktivierungslösung.

Das in dem Saug/Spülkörper enthaltende mindestens eine Superabsorbierende Polymer wird durch die Aktivierung zumindest teilweise in einen gequollenen Zustand versetzt.

Es wurde gefunden, dass der aktivierte Saug-/Spülkörper, wenn er für 24 h einem Unterdruck von 125 mmHg ausgesetzt ist, in einem feuchten gequollenem Zustand verbleiben kann, wobei sich sein Volumen und sein Gewicht verkleinern.

Unter einem volumenstabilem Saug/Spülkörper wird deshalb in diesem Zusammenhang ein aktivierter Saug-/Spülkörper verstanden, welcher mindestens ein Superabsorbierendes Polymer enthält, wobei das Volumen des aktivierten Saug-/Spülkörpers bei einer Temperatur von 20°C während 24 h bei einem Unterdruck von 125 mmHg um mindestens 3 % und höchstens 50 %, vorzugsweise um mindestens 10 % und höchstens 35 % abnimmt. Eine Vorrichtung zur Messung der Volumenstabilität des aktivierten Saug-/Spülkörpers ist in Beispiel 7 der Anmeldung beschrieben.

Weiterhin wurde gefunden, dass der volumenstabile aktivierte Saug/Spülkörper während einer Unterdruckbehandlung von Wunden Aktivierungslösung abgeben und ggf. im Austausch Wundexsudat aufnehmen kann. Dabei weist der volumenstabile aktivierte Saug/Spülkörper während der Unterdruckbehandlung in der Wunde typischerweise eine Volumenabnahme bezogen auf das Volumen zu Beginn der Unterdruckbehandlung von mindestens 1% und höchstens 50 % auf.

Ein Vorteil beim Einsatz eines volumenstabilen Saug-/Spülkörper in der Unterdrucktherapie von Wunden besteht darin, dass während der Unterdruckbehandlung stets eine gleichmäßige Druckverteilung gewährleistet werden kann. Ungleichmäßige Druckverteilung kann sich für den Patienten unangenehm anfühlen und die Wundheilung stören. Es wurde herausgefunden, dass eine Ursache für ungleichmäßige Druckverteilung bei der Verwendung herkömmlicher Absorptionskörper in einer Volumenzunahme bestehen könnte, insbesondere wenn sich diese durch die Volumenzunahme der Kreisform annähern.

Der volumenstabile Saug-/Spülkörpers stellt während der gesamten Behandlung eine weiche Wundauflage in Unterdruckbereichen von bis zu 250 mm Hg bereit.

Ein weiterer Vorteil beim Einsatz eines volumenstabilen Saug-/Spülkörper in der Unterdrucktherapie von Wunden besteht darin, dass sich volumenstabile Saug-/Spülkörper in hervorragender Weise zum Austamponieren des Wundraumes eignen. Da während der Unterdrucktherapie keine Volumenzunahme des Saug-/Spülkörpers erfolgt, bleibt die Wunde während der Behandlung gleichmäßig austamponiert und es bilden sich keine unerwünschten Hohlräume.

Infolge der Volumenabnahme des volumenstabilen Saug-/Spülkörpers während der Unterdrucktherapie erfolgt eine Druckentlastung des Wundnahbereiches mit der Zeit. Dieser weitere positive Effekt, welcher bei Verwendung der erfindungsgemäßen Vorrichtung auftreten kann, hat sich als sehr vorteilhaft für die Wundheilung erwiesen.

Darüber hinaus hat es sich als besonders vorteilhaft erwiesen, dass bei Verwendung eines volumenstabilen Saug-/Spülkörpers bereits unmittelbar nach Beginn der Unterdrucktherapie ein hydroaktiver Effekt vorliegen kann. Zur Untersuchung der Ursache des typischerweise bereits unmittelbar nach Beginn der Behandlung einsetzenden hydroaktiven Effekts wurden Experimente an einem Wundsimulator durchgeführt. Ein geeigneter Wundsimulator ist in der der DE 102008064510.9 beschrieben. Bei den Versuchen wurde beobachtet, dass bereits unmittelbar nach dem Anlegen des Unterdruckverbandes Aktivierungslösung aus dem aktivierten Saugspülkörpers austreten kann. Die Menge der pro Zeiteinheit austretenden Aktivierungslösung kann sehr schnell, beispielsweise innerhalb der ersten drei Stunden, ein Maximum erreichen und dann zurückgehen. Die Menge und Geschwindigkeit der ausgetretenen Aktivierungslösung variiert in Abhängigkeit von Parametern wie insbesondere Stärke des Unterdrucks, aber auch beispielsweise Zusammensetzung Größe, Form, und falls vorhanden der Umhüllung des aktivierten Saug-/Spülkörpers. Der hydroaktive Effekt kann bei intermittierender Unterdruckeinstellung verstärkt werden, da der Saug-/Spülkörpers in Phasen verringerten Unterdrucks mehr Feuchtigkeit an die Wunde abgeben kann. Insbesondere kann bei der Verwendung eines volumenstabilen Saug-/Spülkörpers durch eine Variation des Unterdrucks während der Behandlung eine vorteilhafte Spülwirkung erzielt werden. Dies ermöglicht die Reinigung der Wunde während der Anwendung.

Versuche am Wundsimulator haben weiterhin ergeben, dass ein erster Anteil der aus dem aktivierten Saug-/Spülkörper ausgetretenen Aktivierungslösung von dem Unterdrucksystem abgesaugt und damit aus dem Wundraum irreversibel entfernt wird. Ein zweiter Anteil der ausgetretenen Aktivierungslösung dagegen verbleibt zumindest vorübergehend im Wundraum und könnte für den beobachteten hydroaktiven Effekt verantwortlich sein.

Ein weiterer Vorteil beim Einsatz eines volumenstabilen Saug-/Spülkörper in der Unterdrucktherapie von Wunden besteht darin, dass ein volumenstabiler Saug-/Spülkörper weniger zum Verkleben und /oder Verwachsen mit dem Wundgrund neigt. Dies könnte darin begründet sein, dass der auf den Wundgrund ausgeübte Druck während der Behandlung gleich bleibt oder abnimmt. Dies ist vorteilhaft, da keine zusätzliche Reinigung der Wunde nach dem Abnehmen des Unterdruckverbandes erfolgen muss und der Verbandswechsel deshalb schonender und schneller durchgeführt werden kann. Weiterhin kann eine Traumatisierung der Wunde beim Verbandswechsel vermieden werden, was die Wirksamkeit der Behandlung erhöht.

In überraschender Weise kann bei Verwendung des volumenstabilen Saug-/Spülkörper in der Unterdrucktherapie zudem der ungehinderte Abfluss von Wundexsudat aus des Vorrichtung besser gewährleistet werden. Dieser vorteilhafte Effekt könnte darauf zurückzuführen sein, dass Abflusskanäle im Saug-/Spülkörper oder in einer ggf. zusätzlich vorhandenen Wundkontaktschicht offen gehalten werden. Dagegen könnten Abflusskanäle bei der Verwendung von aus dem Stand der Technik bekannten Absorbtionskörpern, bei denen sich während der Behandlung das Volumen vergrößert, zusammengedrückt und damit teilweise inaktiviert werden.

Die erfindungsgemäße Vorrichtung umfasst ein Abdeckmaterial zum luftdichten Verschließen der Wunde. Unter "luftdichten Verschließen" soll hier nicht verstanden werden, dass keinerlei Gasaustausch zwischen dem Wundraum und seiner Umgebung stattfindet. Vielmehr ist mit "luftdichten Verschließen" in diesem Zusammenhang gemeint, dass unter Berücksichtigung der eingesetzten Unterdruckpumpe der für die Unterdrucktherapie von Wunden nötige Unterdruck aufrechterhalten werden kann. Es können deshalb auch Abdeckmaterialien eingesetzt werden, welche eine geringfügige Gas-Permeabilität aufweisen, so lange der für die Unterdrucktherapie notwendige Unterdruck aufrechterhalten werden kann.

Das luftdichte Abdeckmaterial kann beispielsweise in Form einer aus einem festen Material bestehenden Schale oder in Form einer flexiblen Folie vorliegen. Vorstellbar sind auch Kombinationen von festen Rahmen oder Auflageplatten mit flexiblen Folien.

In einer bevorzugten Ausführungsform der Erfindung umfasst das Abdeckmaterial zum luftdichten Verschließen der Wunde ein wasserunlösliches Polymer, oder eine Metallfolie.

In einer besonders bevorzugten Ausführungsform der Erfindung handelt es sich bei dem wasserunlöslichen Polymer um Polyurethan, Polyester, Polypropylen, Polyethylen, Polyamid oder Polyvinylchlorid, Polyorganosiloxan (Silikon), oder um eine Mischung daraus.

Dem Fachmann sind weitere geeignete polymere Folienmaterialien bekannt.

Als Abdeckmaterial können auch Fertigprodukte eingesetzt werden, welche die vorstehend genannten Eigenschaften aufweisen.

Als besonders geeignetes Abdeckmaterial für die erfindungsgemäße Vorrichtung hat sich Polyurethanfilm der Marke Hydrofilm^{®} (Paul Hartmann AG, Deutschland) oder Visulin ^{®} (Paul Hartmann AG, Deutschland) erwiesen.

Das Abdeckmaterial wird in der Wundumgebung oder am Wundrand so befestigt, dass ein luftdichter Wundverschluss gewährleistet ist. Dabei kann es zweckmäßig sein, wenn das Abdeckmaterial vollflächig selbstklebend ausgestattet ist oder einen selbstklebenden Rand aufweist. Alternativ können Befestigung und Abdichtung beispielsweise mit einer Klebefolie, mit einem flüssigen Kleber oder mit einer Abdichtmasse erfolgen.

In einer bevorzugten Ausführungsform der Erfindung umfasst das Abdeckmaterial eine Folie aus einem oder mehreren wasserunlöslichen Polymeren, wobei die Folie vollflächig selbstklebend ausgestattet ist oder einen selbstklebenden Rand aufweist.

Möglich ist jedoch auch, dass das Abdeckmaterial lediglich durch den während der Unterdruckbehandlung erzeugten Unterdruck an der Wunde gehalten wird.

In einer bevorzugten Ausführungsform werden Abdeckmaterial und das Mittel zur funktionellen Verbindung des Wundraums mit einer außerhalb des Abdeckmaterials befindlichen Unterdruckquelle bereits gebrauchsfertig miteinander verbunden zur Verfügung gestellt. Es ist ganz besonders bevorzugt, dass diese Ausführungsform eine Folie aus einem oder mehreren wasserunlöslichen Polymeren enthält, welche einen selbstklebenden Rand aufweist, da diese Anordnung das Anlegen des Verbandes wesentlich erleichtert.

Die erfindungsgemäße Vorrichtung zur Unterdrucktherapie umfasst ein Mittel zur funktionellen Verbindung des Wundraums mit einer außerhalb des Abdeckmaterials befindlichen Unterdruckquelle, so dass ein Unterdruck im Wundraum herstellbar ist und Flüssigkeiten aus dem Wundraum absaugbar sind.

Der Ausdruck "Unterdruck im Wundraum" bezeichnet im Zusammenhang mit der Erfindung einen innerhalb des Wundverbandes gegenüber dem Umgebungsluftdruck (atmosphärischer Luftdruck) erniedrigten Luftdruck. Unter "innerhalb des Wundverbandes" wird der durch das Abdeckmaterial und der Wunde gebildete Zwischenraum verstanden. "Unterdruck" wird häufig auch als "negativer Druck" bezeichnet.

Die Druckdifferenz zwischen dem Luftdruck innerhalb des Wundverbandes und dem Umgebungsluftdruck wird im Zusammenhang mit der Erfindung in mm Hg (Millimeter-Quecksilbersäule) angegeben, da dies im Bereich der Unterdrucktherapie üblich ist. 1 mm Hg entspricht einem Torr beziehungsweise 133,322 Pa (Pascal). Im Zusammenhang mit der Erfindung wird der Unterdruck, d.h. die Druckdifferenz zwischen dem Luftdruck innerhalb des Wundverbandes und dem Umgebungsluftdruck, als positiver Zahlenwert in mm Hg angegeben.

In einer Ausführungsform der Erfindung handelt es sich bei dem Unterdruck um einen Unterdruck von höchstens 250 mmHg. Dieser Unterdruckbereich von höchstens 250 mmHg hat sich als für die Wundheilung geeignet erwiesen. In einer bevorzugten Ausführungsform der Erfindung handelt es sich bei dem Unterdruck um einen Unterdruck von mindestens 10 mm Hg und höchstens 150 mm Hg.

In zwei weiteren alternativen, jeweils bevorzugten Ausführungsformen der Erfindung handelt es sich bei dem Unterdruck a) um einen konstanten Unterdruck oder b) um einen zeitlich variierenden Unterdruck.

Unter "konstantem Unterdruck" (a) wird hier verstanden, dass der Unterdruck während der Behandlung im Wesentlichen konstant gehalten wird. "Im Wesentlichen konstant" bedeutet, dass während der Behandlung geringfügige Veränderungen des Unterdrucks, beispielsweise um 15 % nach oben oder unten, auftreten können.

Ein bevorzugter konstanter Unterdruck ist der Bereich von mindestens 80 mm Hg bis höchstens 150 mm Hg.

Unter "zeitlich variierendem Unterdruck" (b) wird hier verstanden, dass der Unterdruck während der Behandlung gezielt variiert wird. Unter der gezielten Variation des Luftdrucks werden diejenigen Variationen des Luftdrucks verstanden, die einsetzen, wenn nach Anlegen des Unterdruckverbandes ein erster Sollwert für den Unterdruck erreicht wurde. Dagegen ist die erste Anstiegsphase des Unterdrucks, die nach dem Anlegen des Verbandes bis zum Erreichen des ersten Sollwertes auftritt, nicht von dem Begriff "zeitlich variierender Unterdruck" umfasst. Dies gilt analog für die am Ende der Behandlung nötige Absenkung des Luftdrucks auf den Umgebungsluftdruck, die gleichfalls nicht von dem Begriff "zeitlich variierender Unterdruck" umfasst ist.

Der "zeitlich variierende Unterdruck" ist durch den Umgebungsluftdruck nach unten und durch einen maximalen Unterdruck von 250 mm Hg, vorzugsweise 150 mm Hg, insbesondere 125 mm Hg, nach oben begrenzt. Der während der Behandlung applizierte tatsächliche Unterdruck bewegt sich innerhalb dieser durch ihre Eckwerte definierten Spanne. Der "zeitlich variierende Unterdruck" umfasst also beispielsweise einen ein- oder mehrmaligen Wechsel zwischen einem oder mehreren höheren Unterdruckwerten und einem oder mehreren geringeren Unterdruckwerten. Ebenso umfasst der "zeitlich variierende Unterdruck" einen während der Behandlung erfolgenden gezielten ein- oder mehrmaligen Wechsel zwischen dem Umgebungsdruck und einem oder mehreren höheren Unterdruckwerten.

Bei einer bevorzugten Ausführungsform beträgt der maximale Unterdruck für einen zeitlich variierenden Unterdruck 125 mm Hg. Die untere Grenze für die Variation des Unterdrucks stellt bei dieser Ausführungsform der Umgebungsluftdruck dar. Während der Behandlung variiert der Unterdruck zwischen oder innerhalb dieser Eckwerte.

Bei einer weiteren bevorzugten Ausführungsform beträgt der maximale Unterdruck für einen zeitlich variierenden Unterdruck 125 mm Hg. Die untere Grenze für die Variation des Unterdrucks beträgt bei dieser Ausführungsform 20 mm Hg. Während der Behandlung variiert der Unterdruck zwischen oder innerhalb dieser Eckwerte.

Bei beiden vorgenannten Ausführungsformen kann der Wechsel zwischen dem oberen und dem unterem Druckwert periodisch oder nicht-periodisch erfolgen. Ein periodischer Wechsel ist bevorzugt. Die Zeitintervalle, in denen der höhere Unterdruck und in denen der geringere Unterdruck oder Umgebungsdruck gehalten wird können jeweils unterschiedlich lang sein. Vorzugsweise wird ein geringerer Unterdruck länger gehalten als ein höherer Unterdruck. Geeignete Zeitintervalle, in denen jeweils eine bestimmte Unterdruckeinstellung oder der Umgebungsdruck gehalten wird sind beispielsweise 1 min, 2 min, 5 min, 10 min, 30 min, 1 h, 12h oder 24h.

Besonders bevorzugt ist ein variierender Unterdruck mit den nachstehend genannten Parametern, wobei während der Behandlung kontinuierlich in den angegebenen Zeitabständen zwischen den beiden Unterdruckwerten gewechselt wird:
Ein Unterdruck von 125 mm Hg während 2 min,
danach
ein Unterdruck von 20 mm Hg während 5 min.

Die erfindungsgemäße Vorrichtung kann weiterhin ein Mittel umfassen, so dass der in der Vorrichtung tatsächlich vorhandene Unterdruck überprüfbar und gegebenenfalls einstellbar ist. Das Mittel kann sich im Wundraum oder an einer anderen geeigneten Stelle befinden. So ist es beispielsweise möglich, einen Drucksensor in der Unterdruckleitung zwischen Wundverband und der Unterdruckquelle anzubringen.

Die erfindungsgemäße Vorrichtung zur Unterdrucktherapie von Wunden umfasst weiterhin ein Mittel zur funktionellen Verbindung des Wundraums mit einer außerhalb des Abdeckmaterials befindlichen Unterdruckquelle.

Die funktionelle Verbindung kann beispielsweise mit einer Verbindungsleitung oder mit einem Unterdruck-Anschlussstück hergestellt werden. Unterdruck-Anschlussstücke sind dem Fachmann auch unter der Bezeichnung "Port" bekannt.

Bei einer Ausführungsform handelt es sich bei dem Mittel zur funktionellen Verbindung des Wundraums mit einer außerhalb des Abdeckmaterials befindlichen Unterdruckquelle um mindestens eine Verbindungsleitung. Die mindestens eine Verbindungsleitung kann durch das Abdeckmaterial hindurchgeführt werden.

Alternativ kann die mindestens eine Verbindungsleitung unter dem Rand des Abdeckmaterials durchgeführt werden.

In beiden Fällen ist die Durchtrittsstelle luftdicht abzudichten, so dass der gewünschte Unterdruck im Verband aufrechterhalten werden kann. Als Abdichtmittel eignet sich beispielsweise eine Klebefolie, eine Klebemasse, oder ein Klebestreifen.

Bei der Verbindungsleitung kann es sich beispielsweise um einen Schlauch, um ein Rohr oder um einen anderen Körper mit einem Hohlraum handeln. Ein geeigneter Schlauch ist beispielsweise ein Silicon-Drainageschlauch.

Die Verbindungsleitung verfügt zweckmäßigerweise an dem Ende, welches sich außerhalb des Wundverbandes befindet, über einen Unterdruckadapter, um mit den weiteren Komponenten des Unterdrucksystems verbindbar zu sein.

Die Verbindungsleitung verfügt an dem Ende, welches sich innerhalb des Wundverbandes befindet, eine Öffnung.

In einer weiteren Ausführungsform handelt es sich bei dem Mittel zur funktionellen Verbindung des Wundraums mit einer außerhalb des Abdeckmaterials befindlichen Unterdruckquelle um ein Unterdruck-Anschlussstück (Port), welches auf einer der Innen- oder Außenseite des Abdeckmaterials befestigt werden kann, wobei das Abdeckmaterial geeignete Öffnungen aufweist. Auch bei dieser Ausführungsform ist auf eine luftdichte Abdichtung entweder der Durchtrittsöffnung (Port innen) oder der Auflagefläche (Port außen) zu achten. Die Abdichtung kann beispielsweise mit einer Klebefolie, mit einer Klebemasse, oder mit einem Klebestreifen hergestellt werden. Es ist auch denkbar, dass der Port selbst über entsprechende Befestigungseinrichtungen, wie beispielsweise Klebeflächen, verfügt. Geeignete Unterdruck-Anschlussstücke sind kommerziell erhältlich. Dabei handelt es sich typischerweise um Unterdruck-Anschlussstücke, welche auf Außenseite des Abdeckmaterials befestigt werden.

Auch das Unterdruck-Anschlussstück verfügt zweckmäßigerweise über einen Unterdruckadapter, um mit den weiteren Komponenten des Unterdrucksystems verbindbar zu sein.

In einer bevorzugten Ausführungsform der Erfindung umfasst die Vorrichtung zur Unterdrucktherapie von Wunden mindestens eine Wundauflageschicht zum Einbringen in den Zwischenraum, welcher durch den aktivierten Saug-/Spülkörper und der Wundoberfläche gebildet wird. Die zusätzliche Wundauflageschicht kann mit dem aktivierten Saug-/Spülkörper haftend oder nicht-haftend verbunden sein.

Als Wundauflageschicht kommt grundsätzlich jede aus dem Stand der Technik bekannte Wundauflage in Frage, solange einerseits ein Durchtritt von Wundexsudat durch die Wundauflage hindurch gewährleistet ist und das Material anderseits keine Neigung zum Verwachsen oder Verkleben mit dem Wundgewebe aufweist. Ein Durchtritt von Wundexsudat kann entweder dadurch erfolgen, dass die Wundauflage aus einem für Flüssigkeiten durchlässigem Material gefertigt ist. Dies kann beispielsweise bei Wundauflagen aus einem Schaummaterial der Fall sein. Ein Durchtritt von Wundexsudat kann auch dadurch gewährleistet werden, dass die Wundauflage geeignete Öffnungen oder Kanäle aufweist.

Besonders bevorzugt ist eine Wundauflageschicht aus einem offenzelligen oder halboffenzelligen Schaumstoff, insbesondere aus Polyurethan. Vorteilhaft ist auch die Verwendung von Wundauflagen aus einem strukturiertem Gel, aus einem Hydrokolloid oder aus Polyorganosiloxan (Silikon).

Eine besonders geeignete Wundauflageschicht aus einem strukturierten Gel ist in der Deutschen Patentanmeldungen Az 102008062472.1 offenbart. Dort ist ein Wundverband für die Unterdrucktherapie beschrieben, umfassend eine Trägerschicht sowie eine auf der wundzugewandten Seite der Trägerschicht aufgebrachte Wundkontaktschicht, wobei die Wundkontaktschicht quer zu ihrer flächigen Erstreckung, die Wundkontaktschicht durchdringende Öffnungen aufweist, dadurch gekennzeichnet, dass die wundzugewandte Seite der Wundkontaktschicht Erhebungen und Vertiefungen aufweist und die Wundkontaktschicht lediglich im Bereich der Erhebungen eine Kontaktoberfläche mit einer Hautoberfläche bildet.

Die in den Deutschen Patentanmeldungen Az. DE102008031183.9 und Az. DE102008031182.0 beschriebenen Wundauflagen können in modifizierter Form gleichfalls als Wundauflageschicht für die erfindungsgemäße Vorrichtung verwendet werden. Die notwendige Modifikation besteht darin, dass beispielsweise durch das Einbringen kanalartiger Öffnungen, ein ungehinderter Durchtritt von Wundexsudat ermöglicht wird. Die Wundauflageschichten aus der DE102008031183.9 und der DE 102008031182.0 sind besonders zum Einsatz in der Granulationsphase und in der Epithelisierungsphase geeignet. Sie umfassen einen hydrophilen Polyurethanschaum mit mindestens 10 Gewichtsprozent Wasseranteil.

Geeignete Wundauflagen aus Polyurethan sind als Fertigprodukte kommerziell verfügbar, beispielsweise das Handelsprodukt PermaFoam^{®} der Firma Paul Hartmann AG.

Ebenso kann die Wundauflageschicht eine durchlässige Non-woven-Schicht oder einen Gittertüll umfassen. Die durchlässige Non-woven-Schicht oder der Tüll besteht vorzugsweise aus einem hydrophoben Material, beispielsweise Polyester. Der Tüll kann weiterhin mit einer Salbe ausgestattet sein.

Besonders geeignete Wundauflageschicht sind Salbenkompressen der Marke Hydrotüll^{®} und Atrauman^{®} (Paul Hartmann AG, Deutschland).

Das Problem einer möglichen Verwachsung oder Verklebung der Wundauflage mit dem Wundgewebe wird in einer alternativen Ausführungsform der Erfindung dadurch gelöst, dass die Wundauflage entweder vollständig aus einem bioresorbierbaren Material besteht, oder aber wundseitig mit einem bioresorbierbaren Material ausgestattet ist. Geeignete bioresorbierbare Materialien sind beispielsweise aus der DE19609551 oder aus der WO02/072163 bekannt.

Vorzugsweise, insbesondere zur Verwendung der erfindungsgemäßen Vorrichtung bei der Behandlung infizierter Wunden, weist die Wundauflage eine anti-mikrobielle Beschichtung auf. Vorzugsweise handelt es sich bei der anti-mikrobiellen Beschichtung um eine Silberbeschichtung.

Eine als Wundauflageschicht geeignete Salbenkompresse mit Silberbeschichtung ist das Handelsprodukt Atrauman Ag^{®} (Paul Hartmann AG, Deutschland).

Die Wundauflageschicht kann eine antibiotisch wirksame pharmazeutische Substanz enthalten.

Es kann auch vorgesehen sein, dass die Wundauflageschicht die Wundheilung fördernde Substanzen enthält, die während der Behandlung an die Wundoberfläche abgegeben werden. Ein Beispiel für derartige Substanzen sind Wachstumsfaktoren.

In einer weiteren Ausführungsform umfasst die erfindungsgemäße Vorrichtung mehr als eine Wundauflageschicht, wobei auch Schichten aus unterschiedlichen Materialien umfasst sind. Eine Kombination mehrerer Schichten ermöglicht eine optimale Anpassung an die jeweilige Wundsituation. So kann es beispielsweise vorteilhaft sein, bei einer infizierten Wunde eine Kombination aus einer silberbeschichteten Salbenkompresse mit einem Schaumverband als Wundauflageschicht einzusetzen.

In einer weiteren bevorzugten Ausführungsform umfasst die Vorrichtung zur Unterdrucktherapie von Wunden mindestens eine zusätzliche Druckverteilungsschicht zum Einbringen in den Zwischenraum, welcher durch den aktivierten Saug-/Spülkörper und dem Abdeckmaterial gebildet wird.

Die mindestens eine zusätzliche Druckverteilungsschicht ist unabhängig von der vorstehend genannten Wundkontaktschicht.

Der Vorteil einer zusätzlichen Druckverteilungsschicht kann darin bestehen, dass der durch den Verband auf den Wundgrund ausgeübte Druck durch die Verwendung der Druckverteilungsschicht noch gleichmäßiger verteilbar ist. Weiterhin kann die Druckverteilungsschicht zusätzliches Wundexsudat speichern.

Die zusätzliche Druckverteilungsschicht kann aus einem offenzelligen oder halboffenzelligen Schaumstoff, einem Abstandsgewirke, aus einer Textilschicht, aus einem strukturiertem Gel, oder aus einer durchlässigen Non-woven-Schicht bestehen. Geeignete Textilschichten sind unter anderem ES-Kompressen oder Gittertullen.

Die zusätzliche Druckverteilungsschicht kann so ausgebildet sein, dass Flüssigkeit wie Wundexsudat durch sie hindurchgeleitet wird. Dazu kann die Druckverteilungsschicht geeignete Kanäle oder Öffnung enthalten, oder aus einem für Flüssigkeiten durchlässigem Material bestehen.

Die zusätzliche Druckverteilungsschicht kann mit dem aktivierte Saug-/Spülkörper haftend oder nicht-haftend verbunden sein. Sie kann von dem Saug-/Spülkörper auch durch eine weitere Schicht, beispielsweise ein Lage einer textilen Schicht, getrennt sein.

Weiterhin stellt die Erfindung ein gebrauchsfertiges Set zur Unterdruck-Wundbehandlung, umfassend ein luftundurchlässiges Abdeckmaterial zum luftdichten Verschließen der Wunde und der Wundumgebung, ein Mittel zur funktionellen Verbindung des Wundraums mit einer außerhalb des Abdeckmaterials befindlichen Unterdruckquelle, so dass ein Unterdruck im Wundraum herstellbar ist und Flüssigkeiten aus dem Wundraum absaugbar sind, und mindestens einen aktivierten Saug-/Spülkörper, welcher mindestens ein superabsorbierendes Polymer enthält, zum Einbringen in den Zwischenraum, welcher durch Wundoberfläche und Abdeckmaterial gebildet wird, wobei der aktivierte Saug-/Spülkörper gebrauchsfertig abgepackt vorliegt, zur Verfügung. Der in dem Set enthaltene Saug-/Spülkörper ist wie bei der erfindungsgemäßen Vorrichtung gleichfalls volumenstabil. In einer bevorzugten Ausführungsformen umfasst das Set Abdeckmaterial, Mittel zur funktionellen Verbindung des Wundraums mit einer außerhalb des Abdeckmaterials befindlichen Unterdruckquelle und mindestens einen gebrauchsfertig abgepackten aktivierten Saug-/Spülkörper gemäß Unteranspruch 4, 5 oder 6.

Der vom Set umfasste Saug-/Spülkörpers kann hergestellt werden, indem der trockene Saug-/Spülkörpers durch Befeuchten mit einer Aktivierungslösung, vorzugsweise Ringerlösung, aktiviert und feuchtigkeitsdicht abgepackt wird. Vorzugsweise liegt der aktivierte und gebrauchsfertig abgepackte Saug-/Spülkörpers in steriler Form vor. Idealerweise liegt der aktivierte Saug-/Spülkörpers in steriler, einzeln eingesiegelter gebrauchsfertiger Packung vor. Die Sterilisation kann durch eine Gegendruckdampfsterilisation oder durch andere dem Fachmann als geeignet bekannte Sterilisationsmethoden erfolgen.

Das Set kann weitere optionale Bestandteile wie beispielsweise eine oder mehrere Wundauflageschichten, eine oder mehrere zusätzliche Druckverteilungsschichten, Klebemittel zum Fixieren des Verbands, Abdichtmittel zur Herstellung einer luftundurchlässigen Abdichtung des Verbandes, Drucksensoren, Verbindungselemente für Drucksensoren, zusätzliche Schläuche, Verbindungsstücke für Schläuche, Desinfektionsmittel, Hautpflegemittel, pharmazeutische Zubereitungen oder eine Gebrauchsanweisung enthalten. Es handelt sich bei dem luftundurchlässigen Abdeckmaterial um ein Abdeckmaterial wie zuvor beschrieben.

In einer bevorzugten Ausführungsform umfasst das Set mindestens eine Wundauflageschicht nach Anspruch 8 oder 9.

In einer weiteren bevorzugten Ausführungsform umfasst das Set mindestens eine zusätzliche Druckverteilungsschicht nach einem der Ansprüche 10, 11 oder 12.

Das Set kann auch sowohl die mindestens eine Wundauflageschicht, als auch die mindestens eine zusätzliche Druckverteilungsschicht umfassen.

Vorzugsweise umfasst das Set weiterhin eine gebrauchsfertige Unterdruckeinheit. Die Unterdruckeinheit kann Komponenten wie beispielsweise eine Pumpe, ein oder mehrere Flüssigkeitsbehälter, eine Steuereinheit, eine Stromversorgung, elektrische Anschlussmitteln und Schläuche enthalten. Die Unterdruckeinheit kann auch eine Vorrichtung zur funktionellen Verbindung des Unterdruckverbandes mit einer vorhandenen stationären Unterdruckquelle enthalten.

Vorzugsweise werden alle Komponenten, bei denen dies aus medizinischer Sicht notwendig ist, steril abgepackt zur Verfügung gestellt. Insbesondere wird der aktivierte Saug-/Spülkörper, das Abdeckmaterial, das Mittel zur funktionellen Verbindung des Wundraums mit einer außerhalb des Abdeckmaterials befindlichen Unterdruckquelle, sowie die optional vorhandene Wundauflage oder zusätzliche Druckverteilungsschicht vorzugsweise steril abgepackt zur Verfügung gestellt.

Der Vorteil des gebrauchsfertigen Sets besteht darin, dass der Unterdruckverband schnell, standardisiert und unkompliziert angelegt werden kann. Ein weiterer Vorteil besteht darin, dass alle im Wundbereich eingesetzten Bestandteile des Sets bereits sterilisiert zur Verfügung gestellt werden können.

Weiterhin wird ein Saug-/Spülkörper, welcher mindestens ein Superabsorbierendes Polymer enthält, zur Anwendung in der Unterdrucktherapie von Wunden bereitgestellt, wobei der Saug-/Spülkörper wie in der erfindungsgemäßen Vorrichtung volumenstabil ist. Der aktivierte Saug-/Spülkörper kann in Unterdrucktherapie-Vorrichtungen zur Behandlung von Wunden eingesetzt werden. Besondere Vorteile ergeben sich, wenn es sich bei den Wunden um Brandwunden, um durch mechanische Traumatisierung entstandene Wunden, um eine durch Einwirkung von Chemikalien entstandene Wunde, um durch eine Stoffwechselstörung verursachte Wunden, um durch eine Durchblutungsstörungen verursachte Wunde, oder um durch ein Druckgeschwür verursachte Wunde handelt. Der aktivierte Saug-/Spülkörper als Bestandteil einer Unterdrucktherapie-Vorrichtungen ermöglicht eine schnelle, wirksame und schonende Behandlung von Wunden. Die Vorteile des aktivierten Saug-/Spülkörper beruhen unter anderem auf einer weichen Struktur, auf der Volumenstabilität, auf der verminderten Neigung zum Verkleben und/oder Verwachsen mit dem Wundgrund, und auf der hohen Absorbtionsfähigkeit.

In einer weiteren Ausführungsform wird ein aktivierter Saug-/Spülkörper, welcher mindestens ein Superabsorbierendes Polymer enthält, zur Anwendung in der Unterdrucktherapie bei der Behandlung einer durch eine Hauttransplantation entstandenen Wunde bereitgestellt. Die Anwendung umfasst die Behandlung von durch Spalthaut-Transplantationen und von durch Vollhaut-Transplantationen entstandenen Wunden mittels Unterdrucktherapie. Vorteilhafte Wirkungen ergeben sich durch die weiche Struktur des aktivierter Saug-/Spülkörpers, durch die gleichmäßige Druckverteilung und durch den hydroaktiven Effekt. Bei der Anwendung des aktivierten Saug-/Spülkörpers bei der Behandlung einer durch eine Hauttransplantation entstandenen Wunde kann das Transplantat ("Skin-Graft") ausreichend fixiert und gleichzeitig schädliche Scherkräfte vermieden werden.

Ein weiterer Gegenstand ist ein Verfahren zur Unterdrucktherapie von Wunden, umfassend die Schritte
a) Bereitstellen einer Vorrichtung nach einem der Ansprüche 1 bis 12 oder eines Sets nach Anspruch 13
b) Anlegen des Unterdruckverbandes an der Wunde
c) Herstellung eines Unterdruckes von höchstens 250 mm Hg im Wundraum für mindestens 30 Minuten und höchstens 7 Tage.

### Figuren

Nachstehend wird die erfindungsgemäße Vorrichtung zur Unterdrucktherapie von Wunden anhand von Zeichnungen näher erläutert. Die Erfindung soll jedoch nicht auf die in den Zeichnungen oder in der Beschreibung der Zeichnung dargestellten Ausgestaltungen reduziert verstanden werden. Vielmehr umfasst die erfindungsgemäße Vorrichtung auch Kombinationen der Einzelmerkmale der alternativen Formen.
**Figur 1** schematischer Aufbau der erfindungsgemäßen Vorrichtung zur Unterdrucktherapie von Wunden (Seitenansicht).
**Figur 2** schematischer Aufbau einer Ausführungsform (Seitenansicht).
**Figur 3** schematischer Aufbau einer weiteren Ausführungsform (Seitenansicht)
**Figur 4** schematischer Aufbau einer weiteren Ausführungsform (Seitenansicht)
**Figur 5** schematischer Aufbau einer weiteren Ausführungsform (Seitenansicht)
**Figur 6** schematischer Aufbau einer weiteren Ausführungsform (Seitenansicht)
**Figur 7** schematischer Aufbau einer weiteren Ausführungsform (Seitenansicht)
**Figur 8** schematischer Aufbau einer Versuchsanordnung zur Ermittlung der Volumenstabilität aktivierter Saug-/Spülkörper bei Unterdruck (Seitenansicht)

### Figurenlegende

- 1: Luftundurchlässiges Abdeckmaterial
- 2: Wundoberfläche bzw. Wundgrund
- 3/53: Aktivierter Saug-/Spülkörper, welcher mindestens ein Superabsorbierendes Polymer enthält
- 4: Wundraum
- 5: Wundumgebung (d.h. in der Regel unversehrte Haut)
- 26/36: Unterdruck- Anschlussstück (Port)
- 27/37/47: Verbindungsleitung
- 28/38/48: Sammelgefäß
- 29/39/49: Unterdruckeinheit
- 66: Wundauflageschicht
- 77: Druckverteilungsschicht
- 81: Luftundurchlässiges Abdeckmaterial
- 82: Aktivierter Saug-/Spülkörper, welcher mindestens ein Superabsorbierendes Polymer enthält
- 83: Unterdruck-Anschlussstück (Port)
- 84: Verbindungsleitung
- 85: Sammelgefäß
- 86: Unterdruckeinheit
- 87: Öffnung im luftundurchlässigen Abdeckmaterial
- 88: Platte mit glatter Oberfläche

### Beschreibung der Figuren

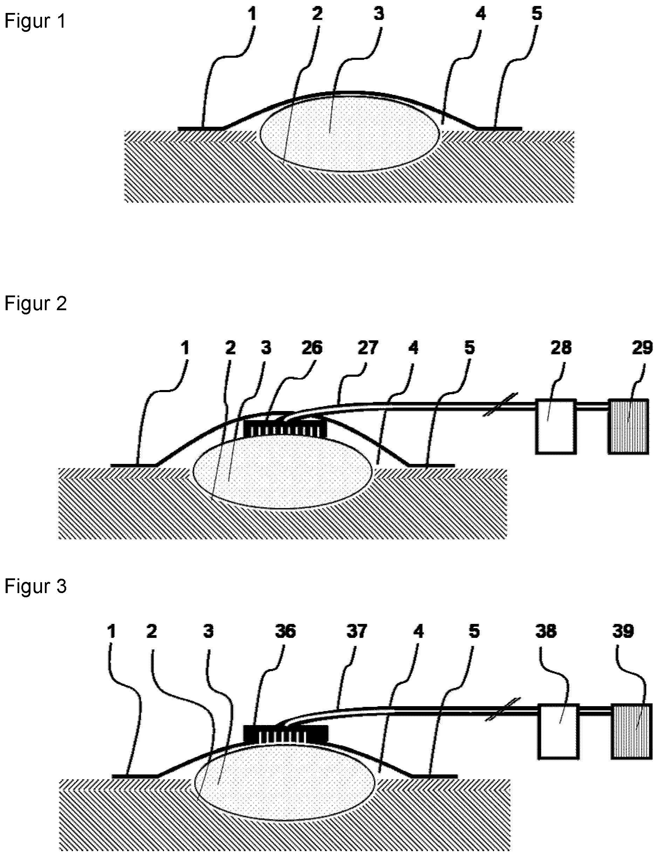

In Figur 1 wird der schematischen Aufbau der erfindungsgemäßen Vorrichtung in der Seitenansicht dargestellt. Die Vorrichtung umfasst ein luftundurchlässiges Abdeckmaterial (1), ein Mittel (in Figur 1 nicht dargestellt) zur funktionellen Verbindung des Wundraums (4) mit einer außerhalb des Abdeckmaterials befindlichen Unterdruckquelle (in Figur 1 nicht dargestellt), sowie mindestens einen aktivierten Saug-/Spülkörper (3), welcher mindestens ein Superabsorbierendes Polymer enthält. Das Abdeckmaterial (1) ist im Bereich der Wundumgebung (5), welche üblicherweise unversehrte Haut aufweist, befestigt. Die Größe des Abdeckmaterials sollte so bemessen sein, dass das Abdeckmaterial außerhalb des Wundbereichs im Bereich der Wundumgebung (5) befestigt werden kann. Das Abdeckmaterial (1) kann in verschiedenen Abmessungen und Formen vorliegen, beispielsweise kreisförmig, oval oder rechteckig. Es kann auch in einer an die Wunde angepassten unregelmäßigen Form vorliegen. Bei dem Abdeckmaterial (1) kann es sich um ein undurchsichtiges Material, um ein teilweise durchsichtiges Material, oder um ein vollständig durchsichtiges Material verwenden. Die Verwendung durchsichtigen Materials kann vorteilhaft sein, um eine Kontrolle des Heilungsverlaufs der Wunde zu ermöglichen. Die Verwendung von nur teilweise durchsichtigem oder undurchsichtigem Material kann vorteilhaft sein, um dem Patienten den Anblick der Wunde zu ersparen. Alternativ kann es sich bei dem Abdeckmaterial (1) auch um ein starres Material, welches in Form einer zur Wunde hin geöffneten Schale über den Wundbereich angebracht und im Bereich der Wundumgebung (5) befestigt wird. Das Abdeckmaterial (1) muss im Bereich der Wundumgebung (5) befestigt und luftdicht abgedichtet werden. Dies kann beispielsweise dadurch erfolgen, dass das Abdeckmaterial (1) einen Kleberand aufweist. Der Kleberand sollte möglichst bis zum Anlegen des Verbandes durch Schutzstreifen geschützt sein. Alternativ kann eine klebende Substanz entweder auf den Rand des Abdeckmaterials (1) und/oder auf die intakte Haut im Bereich der Wundumgebung aufgebracht werden. Dies hat den Vorteil, dass eine Anpassung des Abdeckmaterials an die Form und Größe der Wunde leichter möglich ist. Eine Befestigung und luftdichte Abdichtung der Vorrichtung kann aber auch durch die Verwendung von Klebestreifen oder einer Klebemasse erreicht werden. Figur 1 zeigt weiterhin einen aktivierten Saug-/Spülkörper (3). Beim Anlegen des Verbandes sollte dieser so in den Wundraum (4) eingelegt werden, dass möglichst wenige Hohlräume entstehen und ein guter Kontakt mit dem Wundgrund (2) gewährleistet ist. Hierzu sollten ein oder mehrere aktivierte Saug-/Spülkörper in einer geeigneten Größe ausgewählt werden.

Figur 2 zeigt den schematischen Aufbau einer Ausführungsform der erfindungsgemäßen Vorrichtung in der Seitenansicht. Die Vorrichtung umfasst ein luftundurchlässiges Abdeckmaterial (1), ein Unterdruckanschlussstück (26) zur funktionellen Verbindung des Wundraums (4) über einen Schlauch (27) mit einer außerhalb des Abdeckmaterials befindlichen Unterdruckeinheit (29), sowie mindestens einen aktivierten Saug-/Spülkörper (3), welcher mindestens ein Superabsorbierendes Polymer enthält. Das Unterdruckanschlussstück (26), welches auch als "Port" bezeichnet wird, befindet sich bei der hier gezeigten Ausführungsform auf der zur Wunde weisenden Innenseite des luftundurchlässigen Abdeckmaterials (1). Zwischen Unterdruckanschlussstück (26) und Unterdruckeinheit (29) befindet sich ein Sammelgefäß (28). Bei dieser Ausführungsform muss eine Abdichtung und haftende Befestigung zwischen der Oberseite des Ports und dem Abdeckmaterial gewährleistet werden. Das von der Wunde wegweisende Ende des Ports erstreckt sich typischerweise durch das Abdeckmaterial hindurch und wird mit dem Unterdrucksystem verbunden.

Der schematische Aufbau einer weiteren Ausführungsform der erfindungsgemäßen Vorrichtung wird in Figur 3 in der Seitenansicht dargestellt. Die Vorrichtung unterscheidet sich von der in Fig. 2 gezeigten Vorrichtung dadurch, dass sich das Unterdruck-anschlussstück (36) bei der hier gezeigten Ausführungsform auf der von der Wunde wegweisenden Außenseite des luftundurchlässigen Abdeckmaterials (1) befindet. Um den Wundraum (4) mit einer außerhalb des Abdeckmaterials befindlichen Unterdruckeinheit (39) funktionell zu verbinden, müssen sich bei dieser Anordnung im Bereich des Unterdruckanschlussstücks (36) eine oder mehrere durch das Abdeckmaterial (1) hindurchgehende Öffnungen befinden. Weiterhin ist eine luftdichte Abdichtung zu gewährleisten. Eine solche Abdichtung kann beispielsweise hergestellt werden, indem auf der von der Wunde wegweisenden Oberseite des Ports eine Folie (in Figur 3 nicht gezeigt) aufgebracht wird, die mit dem Abdeckmaterial (1) verklebt wird. Das Anlegen des Verbandes kann erleichtert werden, wenn ein Port verwendet wird, bei ein geeignetes Befestigungs- und Abdichtmittel zum Befestigen des Ports auf dem Abdeckmaterial bereits vorhanden ist. Dies ist beispielsweise bei dem im Handel erhältlichen PPM-Drainageport der Firma Phametra-Pharma und Medica-Trading GmbH (Herne/Ruhrstadt, Deutschland) der Fall.

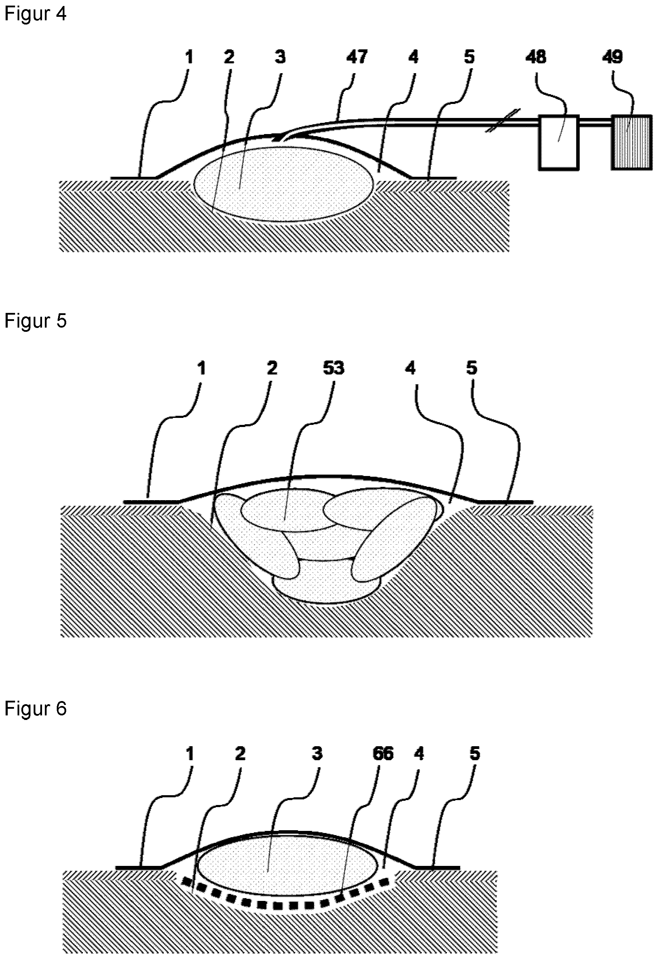

Figur 4 zeigt den schematischen Aufbau einer weiteren Ausführungsform der erfindungsgemäßen Vorrichtung in der Seitenansicht. Auf die Verwendung eines Ports wird bei dieser Ausführungsform verzichtet. Stattdessen wird die funktionelle Verbindung zwischen dem Wundraum (4) und der sich außerhalb des Abdeckmaterials befindlichen Unterdruckeinheit (49) durch einen durch das Abdeckmaterial (1) hindurchgeführten Schlauch (47) hergestellt.

Alternativ kann der Schlauch auch unter dem Rand des Abdeckmaterials hindurchgeführt werden (nicht dargestellt). Eine entsprechende luftdichte Abdichtung ist zu gewährleisten. Der Schlauch weist an seinem Ende, welches sich im Wundraum befindet, eine oder mehrere Öffnungen auf. Die Verwendung eines Schlauches mit einem siebförmigen Endstück, welches mehrere Öffnungen aufweist (nicht dargestellt), kann vorteilhaft sein, da eine Verstopfung des Schlauchs so vermieden werden kann.

Figur 5 zeigt den schematischen Aufbau einer weiteren alternativen Ausführungsform der erfindungsgemäßen Vorrichtung in der Seitenansicht. Die Vorrichtung umfasst ein luftundurchlässiges Abdeckmaterial (1), ein Mittel (nicht dargestellt) zur funktionellen Verbindung des Wundraums (4) mit einer außerhalb des Abdeckmaterials befindlichen Unterdruckquelle (nicht dargestellt), sowie mehr als einen aktivierten Saug-/Spülkörper (53), welcher mindestens ein Superabsorbierendes Polymer enthält. Diese Ausführungsform eignet sich besonders für tiefe oder zerklüftete Wunden. Durch die Verwendung mehrerer aktivierter Saug-/Spülkörper (53) geeigneter Größe, kann eine sehr gute Anpassung an die Form der Wunde erreicht werden. Das Mittel (nicht dargestellt) zur funktionellen Verbindung des Wundraums (4) mit einer außerhalb des Abdeckmaterials (1) befindlichen Unterdruckquelle kann beispielsweise wie in Figur 2 bis 4 dargestellt ausgeführt werden.

Der Einsatz einer Wundauflageschicht (66) in der erfindungsgemäßen Vorrichtung wird in Figur 6 exemplarisch in der Seitenansicht dargestellt. Hierbei handelt sich um eine bevorzugte Ausführungsform der erfindungsgemäßen Vorrichtung. Die Vorrichtung umfasst ein luftundurchlässiges Abdeckmaterial (1), ein Mittel (nicht dargestellt) zur funktionellen Verbindung des Wundraums (4) mit einer außerhalb des Abdeckmaterials befindlichen Unterdruckquelle (nicht dargestellt), mindestens einen aktivierten Saug-/Spülkörper (3), welcher mindestens ein Superabsorbierendes Polymer enthält, sowie eine Wundauflageschicht (66) in dem Zwischenraum, welcher durch den aktivierten Saug-/Spülkörper (3) und der Wundoberfläche (2) gebildet wird. Die Wundauflageschicht (66) sollte so beschaffen sein, dass Wundexudat die Wundauflageschicht (66) passieren kann. Es ist auch möglich mehrere Wundauflageschichten zu verwenden. Dabei können auch unterschiedliche Wundauflageschichten miteinander kombiniert werden. Die Wundauflageschicht (66) kann auch Fasern oder Tamponadestreifen umfassen.

Die zusätzliche Wundauflageschicht (66) kann mit dem aktivierte Saug-/Spülkörper haftend oder nicht-haftend verbunden sein. Sie kann von dem Saug-/Spülkörper auch durch eine weitere Schicht, beispielsweise ein Lage einer textilen Schicht, getrennt sein.

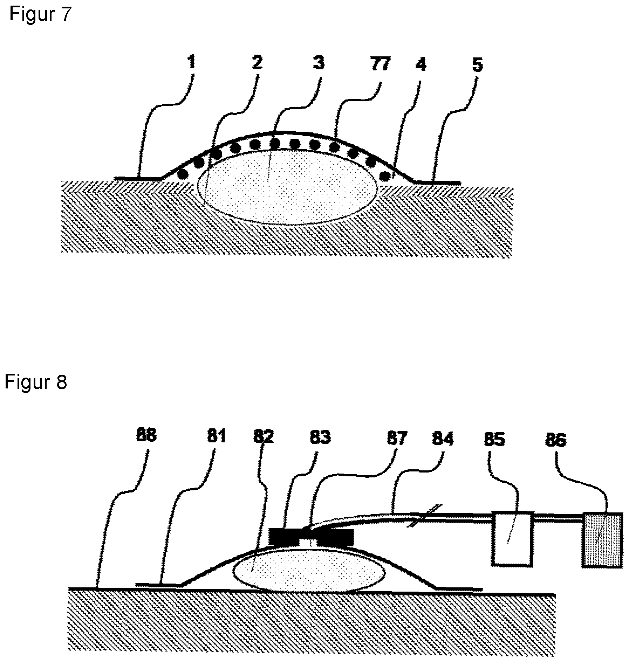

Figur 7 zeigt den schematischen Aufbau einer weiteren bevorzugten Ausführungsform der erfindungsgemäßen Vorrichtung in der Seitenansicht. Diese Ausführungsform zeichnet sich durch die Anwesenheit einer zusätzlichen Druckverteilungsschicht (77) aus. Die Vorrichtung umfasst deshalb ein luftundurchlässiges Abdeckmaterial (1), ein Mittel (nicht dargestellt) zur funktionellen Verbindung des Wundraums (4) mit einer außerhalb des Abdeckmaterials befindlichen Unterdruckquelle (nicht dargestellt), mindestens einen aktivierten Saug-/Spülkörper (3), welcher mindestens ein Superabsorbierendes Polymer enthält, sowie eine Druckverteilungsschicht (77) in dem Zwischenraum, welcher durch den aktivierten Saug-/Spülkörper (3) und dem luftundurchlässigen Abdeckmaterial (1) gebildet wird. Hervorzuheben ist, dass auch eine Kombination der in Figur 6 gezeigten Wundauflageschicht mit der in Figur 7 gezeigten zusätzlichen Druckverteilungsschicht von der Erfindung umfasst ist und eine besonders bevorzugte Ausführungsform darstellt.

Es ist auch möglich mehrere Druckverteilungsschichten zu verwenden, wobei unterschiedliche Druckverteilungsschichten miteinander kombiniert werden können (die Ausführungsformen, welche mehrere Druckverteilungsschichten umfassen sind nicht dargestellt).

Die zusätzliche Druckverteilungsschicht (77) kann so ausgebildet sein, dass Flüssigkeit wie Wundexsudat durch sie hindurchgeleitet wird. Dazu kann die Druckverteilungsschicht geeignete Kanäle oder Öffnung enthalten. Alternativ kann sie aus einem Material bestehen, welches die Durchleitung von Wundexsudat ohne weitere Vorkehrungen zulässt.

Die zusätzliche Druckverteilungsschicht (77) kann so ausgestaltet sein, dass sie auf das Mittel zur funktionellen Verbindung des Wundraums (4) mit einer außerhalb des Abdeckmaterials befindlichen Unterdruckquelle abgestimmt ist. Eine solche Anpassung könnte beispielsweise darin bestehen, dass die Druckverteilungsschicht(en) einen Hohlraum zur Aufnahme des Schlauchendes oder des Ports bereitstellt. Andere Anpassungen sind denkbar.

Die zusätzliche Druckverteilungsschicht kann mit dem aktivierte Saug-/Spülkörper haftend oder nicht-haftend verbunden sein. Sie kann von dem Saug-/Spülkörper auch durch eine weitere Schicht, beispielsweise ein Lage einer textilen Schicht, getrennt sein.

Figur 8 zeigt den schematischen Aufbau einer Versuchsanordnung zur Ermittlung der Volumenstabilität aktivierter Saug-/Spülkörper bei Unterdruck in der Seitenansicht. Die Versuchsanordnung befindet sich auf einer Platte (88) mit glatter, wasserbeständiger Oberfläche und umfasst ein luftundurchlässiges Abdeckmaterial (81), einen aktivierten Saug-/Spülkörper (82), ein Unterdruck-Anschlussstück (83), welches auf der Außenseite des Abdeckmaterials befestigt werden kann, wobei das Abdeckmaterial geeignete Öffnungen (87) aufweist, eine Verbindungsleitung (84), ein Sammelgefäß (85), sowie eine Unterdruckeinheit (86). Das Unterdruck-Anschlussstück (83) befindet sich auf der von der Platte wegweisenden Außenseite des luftundurchlässigen Abdeckmaterials (81).

Zur Messung der Volumenstabilität eines aktivierten Saug-/Spülkörpers bei Unterdruck werden zunächst das Gewicht m₁ und das Volumen V₁ des aktivierten Saug-/Spülkörper bestimmt. Um das Volumen des Saug-/Spülkörpers zu erhalten, wird ein Becherglas mit destilliertem Wasser gefüllt, der Saug-/Spülkörper hinzugefügt und das verdrängte Volumen mittels eines Messzylinders aufgefangen. Der Saug-/Spülkörper muss für diesen Vorgang in eine dünne Plastikfolie gehüllt werden, um eine Flüssigkeitsaufnahme während der Volumenbestimmung zu verhindern. Die Folie wird unmittelbar nach der Volumenbestimmung wieder entfernt. Anschließend wird der aktivierte Saug-/Spülkörper auf die Platte (88) gelegt. Der Saug-/Spülkörper (82) wird dann mit dem luftundurchlässiges Abdeckmaterial (81) vollständig abgedeckt, wobei der Rand des Abdeckmaterial mit der Platte luftdicht verklebt wird. Vorzugsweise handelt es sich bei dem Abdeckmaterial um eine Folie, welche zumindest im Randbereich eine selbsthaftende Oberfläche aufweist. Alternativ kann das Abdeckmaterial beispielsweise mit einem Klebeband luftdicht an der Platte befestigt werden. Anschließend wird in die Mitte des Abdeckmaterial über dem Saug-/Spülkörper ein Loch von etwa 0,5 cm Durchmesser geschnitten. Auf der Außenseite der Folie wird anschließend im Bereich des Lochs ein Unterdruck-Anschlussstück (83) befestigt und luftdicht mit dem Abdeckmaterial verklebt. Das Unterdruck-Anschlussstück wird über eine Verbindungsleitung (84) mit einem Sammelgefäß (85), sowie mit einer Unterdruckeinheit (86) verbunden, so dass ein Unterdruck im dem zwischen Abdeckmaterial (81) und Platte (88) gebildeten Raum herstellbar ist. Anschließend wird für 24 h ein Unterdruck von 125 mm Hg angelegt, wobei darauf zu achten ist, dass die Messapparatur auf einer Temperatur von 20°C (± 3°C) gehalten wird. Während des Versuchs wird dem aktivierten Saug-/Spülkörper keine zusätzliche Flüssigkeit von außen zugeführt. Nach Ablauf der 24 h werden Unterdruck-Anschlussstück und Abdeckmaterial entfernt und das Endgewicht m₂ sowie das Endvolumen V₂ des Saug-/Spülkörper wie vorstehend beschrieben bestimmt. Die absolute Gewichtsveränderung Δm während des Versuchs ergibt sich aus der Differenz m₁ -m₂. Die relative Gewichtsveränderung Δmᵣ bezogen auf das Anfangsgewicht in Prozent ergibt sich nach ((m₁ -m₂) : m₁) x 100). Die absolute Volumenveränderung ΔV während des Versuchs bezogen auf das Anfangsvolumen ergibt sich aus der Differenz V₁ - V₂. Die relative Volumenveränderung ΔVᵣ bezogen auf das Anfangsvolumen in Prozent ergibt sich nach ((V₁ -V₂) : V₁) x 100).

### Beispiele

### Beispiel 1

Die Unterdrucktherapie einer Wunde an einem Patienten wird durchgeführt, indem zunächst ein steriler aktivierter Saug-/Spülkörper (TenderWet^{®}, Paul Hartmann AG, Deutschland) geeigneter Größe in den Wundraum eingebracht wird. Danach wird die Wunde luftdicht mit einer Abdeckfolie (Hydrofilm^{®}, Paul Hartmann AG, Deutschland) verschlossen. Die Befestigung der selbst-haftenden Abdeckfolie erfolgt dabei an der intakten Haut in der unmittelbaren Umgebung der Wunde. In die Abdeckfolie wird mittig ein Loch von ca. 0,5 cm geschnitten. Über dem Loch wird ein PPM-Drainageport^{®} (Phametra-Pharma und Medica-Trading GmbH) aufgelegt und luftdicht befestigt. Der Drainageport wird über einen Schlauch mit dem Unterdrucksystem verbunden. Es wird ein konstanter Unterdruck von 125 mm Hg angelegt. Verbandwechsel und Kontrolle des Heilungsverlaufs erfolgen nach drei Tagen.

### Beispiel 2

Die Unterdrucktherapie einer Wunde erfolgt wie in Beispiel 1 beschrieben. Jedoch wird vor dem Einbringen des Saug-/Spülkörpers eine Lage Atrauman Ag^{®} (Paul Hartmann AG, Deutschland) als Wundkontaktschicht direkt auf den Wundgrund aufgelegt.

### Beispiel 3

Die Unterdrucktherapie einer Wunde erfolgt wie in Beispiel 2 beschrieben. Jedoch wird vor dem Einbringen des Saug-/Spülkörpers eine Lage Hydrotüll^{®} (Paul Hartmann AG, Deutschland) als Wundkontaktschicht direkt auf den Wundgrund aufgelegt.

### Beispiel 4

Die Unterdrucktherapie einer Wunde erfolgt wie in Beispiel 2 beschrieben. Jedoch wird vor der Befestigung der luftdichten Abdeckfolie eine ES-Kompresse (Paul Hartmann AG, Deutschland) als zusätzliche Druckverteilungsschicht direkt auf den Saug-/Spülkörper gelegt.

### Beispiel 5

Unterschiedliche Ausführungsformen der erfindungsgemäßen Vorrichtung wurden an dem in der DE 102008064510.9 beschriebenen Wundsimulator erprobt. Tabelle 1 gibt den Aufbau der Unterdruck-Vorrichtung an. Dabei bedeuten:
a: Abdeckmaterial zum luftdichten Verschließen der Wunde
b: Mittel zur funktionellen Verbindung des Wundraums mit einer außerhalb des Abdeckmaterials befindlichen Unterdruckquelle
c: Optionale Wundauflageschicht, falls vorhanden
d: Optionale zusätzliche Druckverteilungsschicht, falls vorhanden

Bei den nachfolgend angegeben Versuchen wurde als Saug-/Spülkörper ein mit 15 ml Ringerlösung aktivierter TenderWet ^{®} (Paul Hartmann AG, Deutschland) Saug-/Spülkörper (rund, Durchmesser 5,5 cm) cm eingesetzt. Die Versuchsanordnungen wurden jeweils mit konstantem Unterdruck von 125 mm Hg für drei Tage (72 h) bzw. bei einem variierendem Unterdruck (Unterdruck von 125 mm Hg während 2 min, danach ein Unterdruck von 20 mm Hg während 5 min) für drei Tage (72 h) getestet. Der Wundsimulator wurde so eingestellt, dass während 72 h der künstlichen Wunde insgesamt 350 ml Blutersatz (30°C) zugeführt wurde. Der aus der künstlichen Wunde abgesaugte Blutersatz wurde in einem Sammelgefäß gesammelt. Der Blutersatz wurde erhalten, indem eine Mischung aus 425g Glycerin und 566g demineralisiertem Wasser mit 9g NaCl und 0,2 g des Farbstoffs "Allura Red" versetzt wurde.

| Nr | a | b | c | d |
|---|---|---|---|---|
| 1 | Hydrofilm^{®} Transparente PU-Folie (Paul Hartmann AG) | Auf Abdeckfolie befestigter PPM-Drainageport^{®} (Phametra-Pharma und Medica-Trading GmbH) | - | - |
| 2 | Hydrofilm^{®} | Auf Abdeckfolie befestigter PPM-Drainageport^{®} | Atrauman^{®} Ag Silberhaltige Salbenkompresse (Paul Hartmann AG) | - |
| 3 | Hydrofilm^{®} | Auf Abdeckfolie befestigter PPM-Drainageport^{®} | - | ES-Kompresse Mullkompresse (Paul Hartmann AG) |
| 4 | Hydrofilm^{®} | Auf Abdeckfolie befestigter PPM-Drainageport^{®} | Atrauman^{®} Ag | ES-Kompresse |
| 5 | Hydrofilm^{®} | Auf Abdeckfolie befestigter PPM-Drainageport^{®} | Hydrotüll^{®} Hydroaktive Salbenkompresse (Paul Hartmann AG) | - |
| 6 | Hydrofilm^{®} | Auf Abdeckfolie befestigter PPM-Drainageport^{®} | Hydrotüll^{®} | ES-Kompresse |
| 7 | Hydrofilm^{®} | Auf Abdeckfolie befestigter PPM-Drainageport^{®} | PermaFoam^{®} cavity Hydroaktiver PU-Schaumverband (Paul Hartmann AG) | - |
| 8 | Hydrofilm^{®} | Auf Abdeckfolie befestigter PPM-Drainageport^{®} | PermaFoam^{®}cavity | PermaFoam^{®} cavity |
| 9 | Hydrofilm^{®} | Auf Abdeckfolie befestigter PPM-Drainageport^{®} | - | PermaFoam^{®} cavity |
| 10 | Hydrofilm^{®} | Auf Abdeckfolie befestigter PPM-Drainageport^{®} | PermaFoam^{®} cavity | ES-Kompresse |
| 11 | Hydrofilm^{®} | Auf Abdeckfolie befestigter PPM-Drainageport^{®} | - | Zetuvit^{®}-Kompresse Saugkompresse (Paul Hartmann AG) |
| 12 | Hydrofilm^{®} | Auf Abdeckfolie befestigter PPM-Drainageport^{®} | - | Medicomp^{®}-Kom presse Vliesstoffkompresse (Paul Hartmann AG) |
| 13 | Hydrofilm^{®} | Unter Abdeckfolie befestigter PPM-Drainageport^{®} | - | - |
| 14 | Hydrofilm^{®} | Durch Abdeckfolie hindurch geführter Silikonschlauch | - | - |
| 15 | Hydrofilm^{®} | Durch Abdeckfolie hindurch geführter Silikonschlauch | Hydrotüll^{®} | - |
| 16 | Hydrofilm^{®} | Unter Rand der Abdeckfolie hindurch geführter Silikonschlauch | - | - |
| 17 | Tegaderm™ Film Transparente Folie (3M™) | Auf Abdeckfolie befestigter PPM-Drainageport^{®} | - | - |
| 18 | Visulin^{®} Transparente PU-Folie (Paul Hartmann AG) | Auf Abdeckfolie befestigter PPM-Drainageport^{®} | - | - |
| 19 | Hydrofilm^{®} | Auf Abdeckfolie befestigter PPM-Drainageport^{®} | Mepitel^{®} Silikonnetzverband (Mölnlycke Health Care GmbH) | - |
| 21 | Hydrofilm^{®} | Auf Abdeckfolie befestigter PPM-Drainageport^{®} | PPM-Wundschaum grobporig PU-Schaum (Phametra-Pharma und Medica-Trading GmbH) | - |
| 22 | Hydrofilm^{®} | Auf Abdeckfolie befestigter PPM-Drainageport^{®} | PPM-Wundschaum feinporig PU-Schaum (Phametra-Pharma und Medica-Trading GmbH) | - |
| 23 | Hydrofilm^{®} | Auf Abdeckfolie befestigter PPM-Drainageport^{®} | PPM-Wundschaum grobporig | PPM-Wundschaum grobporig |

### Beispiel 6

Bei einem Versuch gemäß Nr. 5 aus Beispiel 5 wurde das Anfangsgewicht m₁ und das Endgewicht m₂ des Tenderwet Active^{®} Saug-/Spülkörpers nach 72 h bestimmt. Während des Versuchs wurde ein konstanter Unterdruck von 125 mm Hg gehalten.

Das Anfangsgewicht m₁ betrug 18,6 g, das Endgewicht m₂ betrug 14,3 g. Dies entspricht einer relativen Gewichtsveränderung Δmᵣ von 23 %.

### Beispiel 7

Die Volumenstabilität eines erfindungsgemäßen, mit 40 ml Ringerlösung aktivierten Saug-/Spülkörpers (Tenderwet ^{®}; 7,5cm x 7,5cm, Trockengewicht vor Aktivierung 3,48 g) wurde bei 125 mm Hg Unterdruck gemessen, wobei die Messapparatur während des Versuchs auf ca. 20°C gehalten wurde. Während des Versuchs wurde dem aktivierten Saug-/Spülkörper keine zusätzliche Flüssigkeit von außen zugeführt. Als Abdeckmaterial wurde eine selbsthaftende Hydrofilm^{®}-Folie (Paul Hartmann AG, Deutschland) verwendet. Weiterhin wurde ein PPM-Drainageport® (Phametra-Pharma und Medica-Trading GmbH) als Unterdruck-Anschlussstück eingesetzt. Als Unterdruckquelle diente eine Atmos S04-Pumpe (Atmos MedizinTechnik GmbH & Co. KG, Lenzkirch, Deutschland).

Der aktivierte TenderWet ^{®} Saug-/Spülkörper wies ein Anfangsgewicht m₁ von 43,46 g und ein Anfangsvolumen V₁ von 41 cm³ auf. Nach 24 h Unterdruck wies der Saug-/Spülkörpers ein Endgewicht m₂ von 25,06 g und ein Endvolumen V₂ von 32 cm³ auf.

Dies entspricht einer relativen Gewichtsveränderung Δmᵣ von 42,34 %, sowie einer relativen Volumenveränderung ΔVᵣ von 21,95 %.

Die aktivierte Saug-/Spülkörpers wies zu Beginn des Versuchs eine feuchte und sehr weiche Oberfläche auf. Diese Eigenschaft blieb über den Versuchszeitraum (24 Stunden) unverändert erhalten.

## Patentansprüche

1. Vorrichtung zur Unterdrucktherapie von Wunden umfassend,
a) ein luftundurchlässiges Abdeckmaterial (1) zum luftdichten Verschließen der Wunde und der Wundumgebung (5)
b) ein Mittel zur funktionellen Verbindung des Wundraums (4) mit einer außerhalb des Abdeckmaterials befindlichen Unterdruckquelle (29, 39, 49), so dass ein Unterdruck im Wundraum (4) herstellbar ist und Flüssigkeiten aus dem Wundraum (4) absaugbar sind
c) mindestens einen aktivierten Saug-/Spülkörper (3, 53), welcher mindestens ein Superabsorbierendes Polymer enthält und welcher mittels einer wässrigen Aktivierungslösung mit einem Wassergehalt von mindestens 50 Gewichtsprozent aktiviert wurde, zum Einbringen in den Zwischenraum, welcher durch Wundoberfläche (2) und Abdeckmaterial (1) gebildet wird, wobei der aktivierte Saug-/Spülkörper (3, 53) mindestens 500 Gewichtsprozent, bezogen auf das Gewicht des trockenen Saug-/Spülkörpers, der wässrigen Aktivierungslösung enthält und wobei es sich bei dem aktivierten Saug-/Spülkörper (3, 53) um einen volumenstabilen Saug-/Spülkörper handelt, so dass der Saug-/Spülkörper (3, 53) während der Unterdruckbehandlung keine Volumenzunahme, sondern eine Volumenabnahme aufweist, wobei die Volumenabnahme mit einer Abnahme des Gewichtes des Saug-/Spülkörpers (3, 53) einhergeht.

2. Vorrichtung zur Unterdrucktherapie von Wunden nach Anspruch 1,
wobei es sich bei dem Superabsorbierenden Polymer um Partikel oder um Fasern handelt.

3. Vorrichtung zur Unterdrucktherapie von Wunden nach einem der vorangehenden Ansprüche, wobei es sich bei dem Superabsorbierenden Polymer um ein Superabsorbierendes Polyacrylat handelt.

4. Vorrichtung zur Unterdrucktherapie von Wunden nach einem der vorangehenden Ansprüche, wobei es sich bei dem Superabsorbierenden Polymer um ein Partikelgemisch von Polyacrylatpartikeln unterschiedlicher Größe handelt, **dadurch gekennzeichnet, dass** das Partikelgemisch
a) 5 bis 100 Gewichtsprozent Partikel mit einer Partikelgröße x mit x ≤ 300 µm und
b) 0 bis 95 Gewichtsprozent Partikel mit einer Partikelgröße x mit x > 300 µm enthält.

5. Vorrichtung zur Unterdrucktherapie von Wunden nach einem der vorangehenden Ansprüche, wobei es sich bei dem aktivierten Saug-/Spülkörper (3, 53) um einen von einer textilen Hülle umgebenen Saug-/Spülkörper handelt.

6. Vorrichtung zur Unterdrucktherapie von Wunden nach einem der vorangehenden Ansprüche, wobei es sich bei dem aktivierten Saug-/Spülkörper (3, 53) um ein umhülltes Airlaid handelt, welches ein Superabsorbierenden Polyacrylat, Cellulosefasern und Polypropylenfasern umfasst.

7. Vorrichtung zur Unterdrucktherapie von Wunden nach einem der vorangehenden Ansprüche, wobei der aktivierte Saug-/Spülkörper (3, 53) im Laufe der Wundbehandlung eine Volumenabnahme bezogen auf das Volumen zu Beginn der Unterdruckbehandlung von mindestens 1 % und höchstens 50 % aufweist.

8. Vorrichtung zur Unterdrucktherapie von Wunden nach einem der vorangehenden Ansprüche, weiterhin umfassend mindestens eine Wundauflageschicht (66) zum Einbringen in den Zwischenraum, welcher durch den aktivierten Saug-/Spülkörper (3, 53) und der Wundoberfläche (2) gebildet wird.

9. Vorrichtung zur Unterdrucktherapie von Wunden nach Anspruch 8, wobei es sich bei der Wundauflageschicht (66) um eine Polyurethanschicht, um eine Hydrokolloidschicht, um ein strukturiertes Gel, um eine Polyorganosiloxan-Schicht, um eine durchlässige Non-woven-Schicht, oder um eine Gittertulle handelt.

10. Vorrichtung zur Unterdrucktherapie von Wunden nach einem der vorangehenden Ansprüche, weiterhin umfassend mindestens eine zusätzliche Druckverteilungsschicht (77) zum Einbringen in den Zwischenraum, welcher durch den aktivierten Saug-/Spülkörper (3, 53) und dem Abdeckmaterial (1) gebildet wird.

11. Vorrichtung zur Unterdrucktherapie von Wunden nach Anspruch 10, wobei es sich bei der Druckverteilungsschicht (77) um einem offenzelligen oder halboffenzelligen Schaumstoff, um ein Abstandsgewirke, um eine Textilschicht, um ein strukturiertes Gel, oder um eine durchlässige Non-woven-Schicht handelt.

12. Vorrichtung zur Unterdrucktherapie von Wunden nach Anspruch 11, wobei es sich bei der Textilschicht um eine textile Wundkompresse oder um eine Gittertulle handelt.

13. Gebrauchsfertiges Set zur Unterdruck-Wundbehandlung umfassend,
a) luftundurchlässiges Abdeckmaterial (1) zum luftdichten Verschließen der Wunde und der Wundumgebung (5),
b) ein Mittel zur funktionellen Verbindung des Wundraums (4) mit einer außerhalb des Abdeckmaterials befindlichen Unterdruckquelle (29, 39, 49), so dass ein Unterdruck im Wundraum (4) herstellbar ist und Flüssigkeiten aus dem Wundraum (4) absaugbar sind,
c) mindestens einen aktivierten Saug-/Spülkörper (3, 53), welcher mindestens ein Superabsorbierendes Polymer enthält und welcher mittels einer wässrigen Aktivierungslösung mit einem Wassergehalt von mindestens 50 Gewichtsprozent aktiviert wurde, zum Einbringen in den Zwischenraum, welcher durch Wundoberfläche (2) und Abdeckmaterial (1) gebildet wird, wobei der aktivierte Saug-/Spülkörper (3, 53) mindestens 500 Gewichtsprozent, bezogen auf das Gewicht des trockenen Saug-/Spülkörpers, der wässrigen Aktivierungslösung enthält und gebrauchsfertig abgepackt vorliegt und wobei es sich bei dem aktivierten Saug-/Spülkörper (3, 53) um einen volumenstabilen Saug-/Spülkörper handelt, so dass der Saug-/Spülkörper (3, 53) während der Unterdruckbehandlung keine Volumenzunahme, sondern eine Volumenabnahme aufweist, wobei die Volumenabnahme mit einer Abnahme des Gewichtes des Saug-/Spülkörpers (3, 53) einhergeht.

## Claims

1. Apparatus for negative-pressure wound therapy, comprising
a) an air-impermeable covering material (1) for airtight closing of the wound and the wound surroundings (5)
b) a means for functional connection of the wound space (4) to a negative-pressure source (29, 39, 49) situated outside the covering material, making it possible to establish negative pressure within the wound space (4) and to aspirate liquids from the wound space (4)
c) at least one activated absorbing/rinsing body (3, 53) containing at least one superabsorbent polymer and having been activated by means of an aqueous activation solution having a water content of at least 50 per cent by weight, for introduction into the gap formed by wound surface (2) and covering material (1), the activated absorbing/rinsing body (3, 53) containing at least 500 per cent by weight, based on the weight of the dry absorbing/rinsing body, of the aqueous activation solution and wherein the activated absorbing/rinsing body (3, 53) is a volume-stable absorbing/rinsing body, so that the absorbing/rinsing body (3, 53), during the negative-pressure treatment, exhibits not an increase in volume but instead a reduction in volume, the reduction in volume being accompanied by a reduction in the weight of the absorbing/rinsing body (3, 53).

2. Apparatus for negative-pressure wound therapy according to Claim 1,
wherein the superabsorbent polymer is particles or fibres.

3. Apparatus for negative-pressure wound therapy according to either of the preceding claims, wherein the superabsorbent polymer is a superabsorbent polyacrylate.

4. Apparatus for negative-pressure wound therapy according to any of the preceding claims, wherein the superabsorbent polymer is a particle mixture of polyacrylate particles varying in size, **characterized in that** the particle mixture contains
a) 5 to 100 per cent by weight of particles having particle size x, where x ≤ 300 µm, and
b) 0 to 95 per cent by weight of particles having particle size x, where x > 300 µm.

5. Apparatus for negative-pressure wound therapy according to any of the preceding claims, wherein the activated absorbing/rinsing body (3, 53) is an absorbing/rinsing body surrounded by a textile shell.

6. Apparatus for negative-pressure wound therapy according to any of the preceding claims, wherein the activated absorbing/rinsing body (3, 53) is an enclosed airlaid comprising a superabsorbent polyacrylate, cellulose fibres and polypropylene fibres.

7. Apparatus for negative-pressure wound therapy according to any of the preceding claims, wherein the activated absorbing/rinsing body (3, 53) exhibits a reduction in volume, based on the volume at the start of the negative-pressure treatment, of at least 1% and not more than 50% over the course of the wound treatment.

8. Apparatus for negative-pressure wound therapy according to any of the preceding claims, additionally comprising at least one wound dressing layer (66) for introduction into the gap formed by the activated absorbing/rinsing body (3, 53) and the wound surface (2).

9. Apparatus for negative-pressure wound therapy according to Claim 8, wherein the wound dressing layer (66) is a polyurethane layer, a hydrocolloid layer, a structured gel, a polyorganosiloxane layer, a permeable nonwoven layer, or a lattice tulle.

10. Apparatus for negative-pressure wound therapy according to any of the preceding claims, additionally comprising at least one additional pressure-distribution layer (77) for introduction into the gap formed by the activated absorbing/rinsing body (3, 53) and the covering material (1).

11. Apparatus for negative-pressure wound therapy according to Claim 10, wherein the pressure-distribution layer (77) is an open-cell or semi-open-cell foam, a spacer knit, a textile layer, a structured gel, or a permeable nonwoven layer.

12. Apparatus for negative-pressure wound therapy according to Claim 11, wherein the textile layer is a textile wound compress or a lattice tulle.

13. Ready-to-use set for negative-pressure wound treatment, comprising
a) air-impermeable covering material (1) for airtight closing of the wound and the wound surroundings (5),
b) a means for functional connection of the wound space (4) to a negative-pressure source (29, 39, 49) situated outside the covering material, making it possible to establish negative pressure within the wound space (4) and to aspirate liquids from the wound space (4),
c) at least one activated absorbing/rinsing body (3, 53) containing at least one superabsorbent polymer and having been activated by means of an aqueous activation solution having a water content of at least 50 per cent by weight, for introduction into the gap formed by wound surface (2) and covering material (1), the activated absorbing/rinsing body (3, 53) containing at least 500 per cent by weight, based on the weight of the dry absorbing/rinsing body, of the aqueous activation solution and being prepackaged such that it is ready to use and wherein the activated absorbing/rinsing body (3, 53) is a volume-stable absorbing/rinsing body, so that the absorbing/rinsing body (3, 53), during the negative-pressure treatment, exhibits not an increase in volume but instead a reduction in volume, the reduction in volume being accompanied by a reduction in the weight of the absorbing/rinsing body (3, 53).

## Revendications

1. Dispositif de traitement de plaies par pression négative, comprenant:
a) un matériau de couverture imperméable à l'air (1), pour fermer hermétiquement la plaie et les alentours de la plaie (5),
b) un moyen pour réaliser une liaison fonctionnelle de l'espace de la plaie (4) à une source de pression négative (29, 39, 49) se trouvant à l'extérieur du matériau de couverture, de telle manière qu'une pression négative puisse être établie dans l'espace de la plaie (4) et que des liquides puissent être aspirés hors de l'espace de la plaie (4),
c) au moins un corps d'aspiration/purge activé (3, 53), qui contient au moins un polymère superabsorbant et qui a été activé au moyen d'une solution aqueuse d'activation ayant une teneur en eau d'au moins 50 pour cent en poids, à introduire dans l'espace intermédiaire qui est formé par la surface de la plaie (2) et le matériau de couverture (1), dans lequel le corps d'aspiration/purge activé (3, 53) contient au moins 500 pour cent en poids, rapportés au poids du corps d'aspiration/purge sec, de la solution aqueuse d'activation et dans lequel le corps d'aspiration/purge activé (3, 53) est un corps d'aspiration/purge de volume stable de telle sorte que le corps d'aspiration/purge (3, 53), pendant le traitement par pression négative, ne présente aucune augmentation de volume, mais une diminution de volume, la diminution de volume étant associée à une diminution du poids du corps d'aspiration/purge (3, 53).

2. Dispositif de traitement de plaies par pression négative selon la revendication 1, dans lequel le polymère superabsorbant est constitué de particules ou de fibres.

3. Dispositif de traitement de plaies par pression négative selon l'une quelconque des revendications précédentes, dans lequel le polymère superabsorbant est un polyacrylate superabsorbant.

4. Dispositif de traitement de plaies par pression négative selon l'une quelconque des revendications précédentes, dans lequel le polymère superabsorbant est un mélange de particules composé de particules de polyacrylate de taille différente, **caractérisé en ce que** le mélange de particules contient
a) 5 à 100 pour cent en poids de particules présentant une taille de particules x avec x ≤ 300 µm et
b) 0 à 95 pour cent en poids de particules présentant une taille de particules x avec x > 300 µm.

5. Dispositif de traitement de plaies par pression négative selon l'une quelconque des revendications précédentes, dans lequel le corps d'aspiration/purge activé (3, 53) est un corps d'aspiration/purge entouré d'une enveloppe textile.

6. Dispositif de traitement de plaies par pression négative selon l'une quelconque des revendications précédentes, dans lequel le corps d'aspiration/purge (3, 53) est un airlaid enveloppé, qui comprend un superabsorbant polyacrylate, des fibres de cellulose et des fibres de polypropylène.

7. Dispositif de traitement de plaies par pression négative selon l'une quelconque des revendications précédentes, dans lequel le corps d'aspiration/purge activé (3, 53) présente, au cours du traitement de la plaie, une diminution de volume d'au moins 1 % et au plus 50 %, par rapport au volume au commencement du traitement par pression négative.

8. Dispositif de traitement de plaies par pression négative selon l'une quelconque des revendications précédentes, comprenant en outre au moins une couche d'appui de plaie (66) à introduire dans l'espace intermédiaire, qui est formé par le corps d'aspiration/purge (3, 53) et la surface de la plaie (2).

9. Dispositif de traitement de plaies par pression négative selon la revendication 8, dans lequel la couche d'appui de plaie (66) est une couche de polyuréthane, une couche hydrocolloïde, un gel structuré, une couche de polyorganosiloxane, une couche non-tissée perméable, ou une tulle à mailles.

10. Dispositif de traitement de plaies par pression négative selon l'une quelconque des revendications précédentes, comprenant en outre au moins une couche supplémentaire de répartition de pression (77), à introduire dans l'espace intermédiaire qui est formé par le corps d'aspiration/purge activé (3, 53) et le matériau de couverture (1).

11. Dispositif de traitement de plaies par pression négative selon la revendication 10, dans lequel la couche de répartition de pression (77) est une mousse à cellules ouvertes ou semi-ouvertes, un tricot d'écartement, une couche textile, un gel structuré, ou une couche non-tissée perméable.

12. Dispositif de traitement de plaies par pression négative selon la revendication 11, dans lequel la couche textile est une compresse de pansement ou une tulle à mailles.

13. Ensemble prêt à l'emploi pour le traitement de plaies par pression négative comprenant
a) un matériau de couverture imperméable à l'air (1), pour fermer hermétiquement la plaie et les alentours de la plaie (5),
b) un moyen pour réaliser une liaison fonctionnelle de l'espace de la plaie (4) à une source de pression négative (29, 39, 49) se trouvant à l'extérieur du matériau de couverture, de telle manière qu'une pression négative puisse être établie dans l'espace de la plaie (4) et que des liquides puissent être aspirés hors de l'espace de la plaie (4),
c) au moins un corps d'aspiration/purge activé (3, 53), qui contient au moins un polymère superabsorbant et qui a été activé au moyen d'une solution aqueuse d'activation ayant une teneur en eau d'au moins 50 pour cent en poids, à introduire dans l'espace intermédiaire qui est formé par la surface de la plaie (2) et le matériau de couverture (1), dans lequel le corps d'aspiration/purge activé (3, 53) contient au moins 500 pour cent en poids rapportés au poids du corps d'aspiration/purge sec de la solution aqueuse d'activation et est emballé prêt à l'emploi et dans lequel le corps d'aspiration/purge activé (3, 53) est un corps d'aspiration/purge de volume stable, de telle sorte que le corps d'aspiration/purge (3, 53), pendant le traitement par pression négative, ne présente aucune augmentation de volume, mais une diminution de volume, la diminution de volume étant associée à une diminution du poids du corps d'aspiration/purge (3, 53).
